# FASCICULE DE BREVET EUROPEEN

(11) **EP 0 672 124 B1**
(45) Date de publication et mention de la délivrance du brevet: **24.07.2002**
(21) Numéro de dépôt: 94901995.4
(22) Date de dépôt: 07.12.1993
(51) Int. Cl.: C12N 9/02, C12N 9/24, C12N 15/53, C12N 15/56, C12N 15/82, C12N 5/10, A01H 5/00, A01N 63/00

(54) **UTILISATION D'UNE SEQUENCE D'ADN CODANT POUR UNE PROTEINE SUSCEPTIBLE DE DEGRADER L'ACIDE OXALIQUE A TITRE DE GENE DE SELECTION**
VERWENDUNG EINER DNA SEQUENZ, KODIERENDE FÜR EIN PROTEIN MIT DER FÄLIGKEIT OXALSÄURE ABZUBAUEN, ZUR GENSELEKTION
USE OF A DNA SEQUENCE CODING FOR AN OXALIC ACID DEGRADING PROTEIN AS A SELECTION GENE

(30) Priorité: 07.12.1992 FR 9214721
(43) Date de publication de la demande: 20.09.1995
(73) Titulaire: Biogemma, 75001 Paris (FR)
(72) Inventeur: PIGNARD, Annie, F-31120 Roquettes (FR); GREZES-BESSET, Bruno, F-31770 Colomiers (FR); GRISON, René, F-31750 Escalquens (FR); SCHNEIDER, Michel, F-31000 Toulouse (FR)
(74) Mandataire: Gillard, Marie-Louise
(86) Numéro de dépôt international: FR9301203
(87) Numéro de publication internationale: WO9413790

(56) Documents cités:
- WO-A-92/01792
- WO-A-92/14824
- WO-A-92/15685
- J. BIOL. CHEM. vol. 266, no. 16 , 5 Juin 1991 , AM. SOC. MOL. BIOL., INC.,BALTIMORE, US; pages 10461 - 10469 B.G. LANE ET AL. 'Homologies between members of the germin gene family in hexaploid wheat and similarities between these wheat germins and certain Physarum spherulins' cité dans la demande

## Description

L'invention concerne une nouvelle utilisation d'une séquence d'ADN codant pour une protéine susceptible de dégrader l'acide oxalique pour sélectionner des cellules végétales, en particulier des cellules végétales ayant intégré un gène d'intérêt et un nouveau procédé pour sélectionner sur acide oxalique des cellules, des cals ou des plantes transformés.

Depuis l'avènement des premières plantes transgéniques en 1983, le nombre de celles-ci a connu une croissance accélérée. Les vecteurs de transformation qui ont été développés à cette époque et qui sont toujours utilisés, tels que par exemple le vecteur pBIN19 (M. Bevan, 1984, Nucl. Ac. Res., 12, 8711-8721) font intervenir, comme gène de sélection des cellules végétales transformées, un gène de résistance à un antibiotique, la kanamycine. L'usage de ce mode de sélection, généralement facile à mettre en oeuvre, bon marché et applicable à de nombreuses espèces végétales, s'est très largement répandu dans les laboratoires de recherche.

Depuis que les premiers essais aux champs, donc hors confinement, de plantes transgéniques ont eu lieu en 1986, l'usage d'un gène de résistance à un antibiotique comme gène de sélection a fait l'objet de nombreuses critiques (cf. notamment F. Casse-Delbart et M. Tepfer, 1990, Biofutur, juin, 56-59 ainsi que J. Bryant et S. Leather, 1992, Tibtech, 10, 274-275). Le risque d'une transmission du gène de résistance de la plante transgénique à une bactérie du sol et, par la suite, à une bactérie potentiellement pathogène pour l'homme, bien qu'étant à priori très faible et encore jamais mis en évidence, n'est pas à négliger (J.A.Heinemann, 1991, TIG, 7, 181-185).

De nombreux substituts au gène de résistance à la kanamycine ont été proposés (M.Ratner, 1989, Bio-Technology, 7, 337-341) mais la plupart font intervenir, soit une résistance à un autre antibiotique (telle que par exemple la gentamycine, la streptomycine, le méthotréxate ou l'hygromycine), soit une résistance à un herbicide (tels que par exemple le bromoxynil ou la phosphoinothricine), ce qui soulève des objections semblables. Une autre approche proposée a été l'élimination après usage du gène de résistance grâce à un système de recombinaison homologue (E.C.Dale et D.W.Ow, 1991, Proc. Natl. Acad. Sci. USA, 88, 10558-10562). Ce système, appelé système cre/lox, présente toutefois le désavantage de nécessiter une transformation ultérieure des plantes transgéniques pour y introduire le gène cre responsable de la recombinaison, suivie d'une autofécondation des plantes afin de pouvoir faire ségréger dans les descendances ce gène cre du gène d'intérêt. Il n'est donc pas simple à utiliser. De plus, ce système laisse présent dans les plantes transgéniques une copie des séquences lox, lesquelles ne présentent aucun intérêt agronomique.

L'invention propose, comme gène de sélection des plantes transgéniques, un gène codant pour une protéine susceptible de dégrader l'acide oxalique, phytotoxine produite par de nombreuses espèces de champignons. Ce gène de sélection qui reste dans les plantes transgéniques présente un intérêt agronomique car il a un effet phytoprotecteur vis-à-vis de ces champignons.

L'invention concerne donc l'utilisation d'une séquence d'ADN codant pour une protéine susceptible de dégrader l'acide oxalique à titre de gène de sélection de cellules végétales.

La protéine susceptible de dégrader l'acide oxalique peut être une enzyme à activité décarboxylase, telle que notamment l'oxalate décarboxylase d'*Aspergillus* ou de *Collybia velutipes* ou de préférence une enzyme à activité oxydase, telle que par exemple l'oxalate oxydase d'orge (commercialisée par Boehringer, réf. 567 698), de sorgho (Chandra S. Pundier, 1991, Phytochemistry, 30, 4, p. 1065) ou de mousse [*Mnium menziesii* (M.F. Laker et al, 1980, Clinical Chemistry, 26, 7, 827)].

Une protéine à activité oxalate oxydase particulièrement appréciée est la protéine de séquence [SEQ ID N°1] : ou de séquence présentant un degré d'homologie élevé avec la séquence [SEQ ID N°1].

La séquence [SEQ ID N°1] est celle de la germine de blé, protéine induite pendant la germination du blé, dont la séquence a été décrite par E. Dratewka-Kos, 1989, J. Biol. Chem, 264, 4896-4900 et B.G. Lane, 1991, J. Biol. Chem., 266, 10461-10469.

Un degré d'homologie élevé signifie ici une homologie (rapport entre les acides aminés identiques et le nombre total d'acides aminés) d'au moins 80 % des séquences d'acides aminés, lorsqu'elles sont alignées d'après l'homologie maximale, selon la méthode d'alignement optimal des séquences de Needleman et Wunsch, 1970, J. Mol. Biol, 48, 443-453. Cette méthode est notamment utilisée dans le logiciel UWGCG de l'Université du Wisconsin : Devereux et al., 1984, Nucl. Ac. Res., 12, 387-395 - option GAP.

Un exemple de protéine présentant un degré d'homologie élevé avec la séquence [SEQ ID N°1] est celle de l'oxalate oxydase d'orge dont la séquence est décrite dans la demande de brevet WO 92/14824 (cette séquence présente une homologie de 96% avec la séquence [SEQ ID N°1]), ou celle d'autres oxalate oxydases de céréales proches du blé.

Compte tenu de la dégénérescence du code génétique il existe un grand nombre de séquences nucléotidiques codant pour l'oxalate oxydase de séquence [SEQ ID N°1]. Parmi celles-ci on apprécie particulièrement la séquence [SEQ ID N°2]

Selon une variante de l'invention, la séquence d'ADN codant pour une protéine susceptible de dégrader l'acide oxalique peut être utilisée en combinaison avec une séquence d'intérêt.

Ainsi selon cette variante, l'invention concerne l'utilisation de la séquence d'ADN codant pour une protéine susceptible de dégrader l'acide oxalique pour sélectionner des cellules végétales transformées avec une séquence d'intérêt, la transformation étant réalisée soit à l'aide de deux vecteurs distincts portant l'un la séquence d'ADN codant pour une protéine susceptible de dégrader l'acide oxalique, l'autre la séquence d'intérêt, soit avec un seul vecteur contenant les deux séquences ci-dessus, ce vecteur étant ci-après dénommé ADN recombinant.

La séquence d'intérêt est toute séquence d'ADN procurant un avantage aux cellules végétales lorsqu'elle est intégrée dans leur génome. Elle peut être par exemple une séquence régulatrice avantageuse. Elle peut être aussi une séquence codant pour une protéine d'intérêt ou pour un précurseur de cette dernière.

Selon un mode de réalisation préféré de l'invention, la séquence d'intérêt confère aux plantes une résistance aux agents pathogènes, tels que les champignons, les bactéries, ainsi que les arthropodes, notamment les insectes et les nématodes.

Une telle séquence d'intérêt peut être par exemple une séquence codant pour une protéine à activité endochitinase ou pour un précurseur de cette dernière. On sait en effet, comme décrit dans la demande de brevet WO 92/01792, qu'une telle protéine a un effet phytoprotecteur car elle est capable de dégrader la chitine, polymère polysaccharidique constitué d'unités N-acétyl-glucosamine associées par des liaisons β-1,4, qui est un composé structural important de la paroi de la plupart des champignons pathogènes, de l'exosquelette des arthropodes, en particulier des insectes, et de l'enveloppe externe des oeufs et des cystes de nématodes.

Une séquence intéressante codant pour une protéine à activité endochitinase ou pour un précurseur de cette dernière, est celle décrite dans la demande de brevet WO 92/01792, qui code pour une protéine comprenant la séquence [SEQ ID N°3] ; cette séquence [SEQ ID N°3] correspond à la séquence [SEQ ID N°1] de la demande WO 92/01792.

On apprécie particulièrement que cette séquence code pour un précurseur d'une protéine à activité endochitinase, qui comprend, en amont de la séquence [SEQ ID N°3], le peptide signal de séquence [SEQ ID N°4] ; ce peptide-signal correspond au peptide signal ayant la séquence [SEQ ID N°3] décrite dans la demande WO 92/01792.

Il est alors avantageux que le peptide signal de séquence [SEQ ID N°4] soit séparé de la protéine à activité chitinase de séquence [SEQ ID N°3], par le peptide de séquence [SEQ ID N°5] ; ce peptide correspond au peptide ayant la séquence [SEQ ID N°2] décrite dans la demande WO 92/01792.

Parmi les nombreuses séquences nucléotidiques qui codent pour un précurseur de la protéine de séquence [SEQ ID N°3] comprenant en amont de celle-ci le peptide signal de séquence [SEQ ID N°4], séparé de la protéine de séquence [SEQ ID N°3] par le peptide de séquence [SEQ ID N°5], la séquence d'ADN [SEQ ID N°6] est particulièrement préférée. Cette séquence, qui correspond à la séquence SEQ ID N°4 décrite dans la demande WO 92/01792, comporte deux introns en position 443-521 et en position 676-756

Une autre séquence codant pour une protéine à activité endochitinase ou pour un précurseur de cette dernière est celle de la chitinase d'*Aphanocladium album* décrite dans la demande EP-A1-531 218 qui comprend la séquence [SEQ ID N°7]. Cette séquence correspond à la séquence [SEQ ID N°1] de la demande EP-A1-531 218.

On apprécie particulièrement que cette séquence code pour un précurseur d'une protéine à activité endochitinase, qui comprend, en amont de la séquence [SEQ ID N°7], le peptide signal de séquence [SEQ ID N°8]. Ce peptide signal correspond au peptide signal ayant la séquence [SEQ ID N°4] de la demande EP-A1-531 218.

Il est alors avantageux que le peptide signal de séquence [SEQ ID N°8] soit séparé de la protéine à activité chitinase de séquence [SEQ ID N°7], par le peptide de séquence [SEQ ID N°9]. Ce peptide correspond au peptide ayant la séquence [SEQ ID N°5] décrite dans la demande EP-A1-531 218.

Parmi les nombreuses séquences nucléotidiques qui codent pour la protéine de séquence [SEQ ID N°7], une séquence particulièrement appréciée est la séquence d'ADN [SEQ ID N°10] qui correspond à la séquence [SEQ ID N°6] décrite dans la demande EP-A1-531 218.

Une autre séquence d'intérêt intéressante qui confère aux plantes une résistance aux agents pathogènes est celle qui code pour une protéine à activité β-1,3-glucanase ou pour un précurseur de cette demière. On sait en effet, comme décrit dans la demande de brevet WO-92 16632, qu'une telle protéine a un effet phytoprotecteur car elle est capable de dégrader les β-1,3-glucanes, polymères polysaccharidiques constitués d'unités glucose associées par des liaisons β-1,3 présentant parfois des ramifications de type β-1,4 ou β-1,6, qui sont un composé structural important de la paroi de la plupart des champignons, et notamment des champignons phytopathogènes.

Une telle séquence avantageuse est celle décrite dans la demande de brevet WO 92/16 632, qui code pour une protéine comprenant la séquence [SEQ ID N° 11]. Cette séquence correspond à la séquence (a₁) décrite dans la demande WO 92/16 632.

Il est intéressant que cette séquence d'intérêt comprenne, immédiatement en aval de la séquence codant pour la séquence [SEQ ID N° 11], la séquence [SEQ ID N° 12] éventuellement tronquée dans sa partie carboxy-terminale de 0 à 27 acides aminés. Cette séquence [SEQ ID N°12] correspond à la séquence (a₄) décrite dans la demande WO 92/16 632.

Cette séquence d'intérêt comprend alors de préférence, immédiatement en amont de la séquence codant pour la séquence [SEQ ID N° 11], un codon CAA ou CAG codant pour Gln.

Une séquence de ce type particulièrement appréciée est celle codant pour une protéine à activité β-1,3-glucanase ou un précurseur de cette dernière qui comprend la séquence [SEQ ID N° 13]. Cette séquence correspond à la séquence (a₅) décrite dans la demande WO 92/16 632.

On apprécie particulièrement que cette séquence code pour un précurseur d'une protéine à activité β-1,3-glucanase qui comprend en amont de la séquence [SEQ ID N° 13], le peptide signal de séquence [SEQ ID N° 14]. Ce peptide signal correspond au peptide signal ayant la séquence (a₂) décrite dans la demande WO 92/16 632.

Parmi les nombreuses séquences nucléotidiques qui codent pour la protéine de séquence [SEQ ID N° 13], une séquence avantageuse est la séquence d'ADN [SEQ ID N° 15] qui correspond à la séquence (Na₁) décrite dans la demande WO 92/16 632.

L'ADN recombinant défini ci-dessus, comprenant le gène codant pour l'oxalate oxydase flanqué des signaux nécessaires à son expression ainsi qu'une séquence d'intérêt, est introduit dans les cellules végétales à transformer. Lorsque la séquence d'intérêt code pour une protéine ou un précurseur de celle-ci, elle comprend également les signaux nécessaires à son expression. La construction contenant ces séquences peut être réalisée dans un vecteur unique ou dans des vecteurs différents qui seront utilisés pour la transformation.

Le promoteur est de préférence un promoteur constitutif fort, par exemple le promoteur 35S du virus de la mosaïque du chou-fleur, ou un promoteur commandant une expression tissu ou organe spécifique comme le promoteur de la petite sous-unité de la ribulose 1,5-bisphosphate carboxylase-oxygénase qui s'exprime préférentiellement dans les feuilles et tout particulièrement les tissus du mésophylle (Kuhlemeier et al., 1987, Ann. Rev. Plant Physiol, 38, 221-257). On peut également utiliser un promoteur spécifique commandant par exemple une expression dans les graines ou au cours d'un stade précis du développement de la plante, ou un promoteur inductible à la suite d'un choc thermique, d'une blessure ou de l'interaction entre la plante et des parasites (Kuhlemeier et al., 1987, référence citée ci-dessus), si une expression de l'ADN recombinant est recherchée dans ces situations.

On utilise la séquence terminatrice, comportant des sites de polyadénylation, pouvant être isolée de gènes végétaux ou de gènes s'exprimant dans les végétaux, comme par exemple le terminateur du gène de la nopaline synthase d'*Agrobacterium tumefaciens.*

Une bactérie, par exemple de l'espèce *Escherichia coli*, qui contient l'ADN recombinant défini ci-dessus avec les moyens permettant sa réplication peut servir au clonage de cet ADN recombinant et une bactérie susceptible d'infecter une plante avec transfert de matériel génétique, par exemple de l'une des espèces *Agrobacterium rhizogenes* et *Agrobacterium tumefaciens*, qui contient cet ADN dans un contexte permettant sa réplication peut servir à transformer des cellules végétales. La transformation des cellules végétales par l'ADN recombinant ci-dessus peut également être effectuée par une autre méthode biologique telle que la voie du tube pollinique (Zhong-xun Luo et al., Plant Molec. Biol. Rep., 1988, 6, 165-176), la transformation directe de graines en germination (Toepfer R. et al., 1989, The Plant Cell., 1, 133-139), ou par une méthode physique telle que l'utilisation de polyéthylèneglycol, de l'électroporation (Chistou P. et al., 1987, Proc. Ntl. Acad. Sci. USA, 84, 3662-3699) ou du bombardement à l'aide de microprojectiles (Klein T.M. et al., 1988, Proc. Ntl. Acad. Sci. USA, 85,8502-8505).

L'invention conceme donc également une cellule végétale, caractérisée en ce qu'elle est transformée par l'ADN recombinant défini précédemment, avec les moyens nécessaires à l'expression de la protéine susceptible de dégrader l'acide oxalique et de la protéine d'intérêt ou du précurseur de cette dernière; une telle cellule peut être sélectionnée sur un milieu contenant de l'acide oxalique. Cette cellule végétale peut provenir d'une espèce de grande culture, telle que par exemple le maïs, le soja, la betterave, le blé, l'orge, le pavot, le colza, le tournesol, la luzerne et le sorgho, d'une espèce florale, telle que le rosier, l'oeillet, le gerbera ou d'une espèce potagère, telle que la carotte, la tomate, la salade, la chicorée, le poivron, le melon et le chou. Des espèces particulièrement appréciées sont le colza *Brassica napus*, le toumesol *Helianthus annuus* et le tabac *Nicotiana tabacum.*

L'étape de transformation qui concerne une ou plusieurs cellules est suivie d'une étape de multiplication de ces cellules transformées de façon à obtenir des cals, lesquels peuvent donner naissance à des plantes transformées par des processus d'organogénèse ou d'embryogénèse.

L'invention conceme donc aussi une plante ou une partie de plante, caractérisée en ce qu'elle contient l'ADN recombinant défini précédemment, avec les moyens nécessaires à l'expression du gène codant pour la protéine susceptible de dégrader l'acide oxalique et du gène codant pour la protéine d'intérêt ou du précurseur de cette demière et en ce qu'elle a été sélectionnée sur un milieu contenant de l'acide oxalique. Une partie de plante particulièrement appréciée est la partie apte à former une nouvelle plante complète, notamment après semis, enfouissement ou repiquage, ou à produire des semences. Une telle partie est par exemple un grain, une graine, une semence, une bouture, une marcotte. Ces plantes peuvent être plus particulièrement des espèces *Nicotiana tabacum, Helianthus annuus* et *Brassica napus.*

La protéine susceptible de dégrader l'acide oxalique peut être une enzyme à activité décarboxylase, telle que notamment l'oxalate décarboxylase d'*Aspergillus* ou de *Collybia velutipes* ou de préférence une enzyme à activité oxydase, telle que par exemple l'oxalate oxydase d'orge (commercialisée par Boehringer, réf. 567 698), de sorgho (Chandra S. Pundier, 1991, Phytochemistry, 30, 4, p. 1065) ou de mousse (*Mnium menziesii*) (M.F. Laker et al, 1980, Clinical Chemistry, 26, 7, 827). Une protéine à activité oxalate oxydase particulièrement appréciée est la protéine de séquence [SEQ ID N°1], ou une séquence présentant un degré d'homologie élevé avec la séquence [SEQ ID N°1]. Celle-ci est avantageusement codée par la séquence d'ADN [SEQ ID N°3].

L'acide oxalique est une phytotoxine produite par de nombreux champignons pathogènes, tels que notamment *Sclerotinia sclerotiorum* (B. Grezes-Besset, 1988, Thèse de Doctorat, Université Paul Sabatier, Toulouse, ainsi que G. Goday et al, 1990, Physiological and Molecular Plant Pathology, 37, 179-191), *Sclerotium rolfsii* (D.F. Bateman et al , 1965, Phytopathology, 68, 1597-1599), *Aspergillus niger* (I.A.S. Gibson, 1953, Transactions British Mycological Society, 36, 198-209), *Cristulariella pyramidalis (P.* Kurian et la, 1979, Phytopathology, 69, 712-714) et *Cryphonectria parasitica* (A.R. Bennett et al, 1990, Mycologia, 358-363).

L'invention a également trait à un procédé pour sélectionner sur acide oxalique des cellules, des cals ou des plantes transformées par un ADN recombinant défini précédemment, caractérisé en ce que, dans le milieu de sélection, le calcium est sous forme soluble.

Les plantes peuvent provenir d'une espèce de grande culture, telle que par exemple le maïs, le soja, la betterave, le blé, l'orge, le pavot, le colza, le tournesol, la luzeme et le sorgho, d'une espèce florale, telle que le rosier, l'oeillet, le gerbera ou d'une espèce potagère, telle que la carotte, la tomate, la salade, la chicorée, le poivron, le melon et le chou. Des espèces particulièrement appréciées sont le colza *Brassica napus*, le tournesol *Helianthus annuus* et le tabac *Nicotiana tabacum.*

Le milieu de sélection comprend de l'acide oxalique et tous les éléments nécessaires à la multiplication et la différentiation des cellules végétales et notamment du calcium indispensable pour leur développement, qui doit donc rester disponible. En présence d'acide oxalique le calcium a tendance à s'associer à ce demier pour former un sel d'oxalate insoluble, ce qui le rend indisponible pour les cellules végétales. II est donc nécessaire que le milieu de sélection contienne des agents permettant de garder le calcium sous forme soluble.

De préférence ces agents sont des agents chelateurs ayant une affinité pour le calcium supérieure à celle de l'acide oxalique. Ceux-ci doivent bien sûr, de plus, ne pas être toxiques pour les cellules.

Des exemples d'agents chélateurs ayant une affinité pour le calcium supérieure à celle de l'acide oxalique sont l'EDTA et l'EGTA. Dans le cas du toumesol, l'EGTA est un agent chélateur particulièrement apprécié.

Ainsi selon un autre aspect, l'invention a pour objet un procédé pour sélectionner sur acide oxalique des cals ou des plantes transformées par une séquence d'ADN codant pour une protéine susceptible de dégrader l'acide oxalique, qui consiste à cultiver les cals ou les plantes sur un milieu contenant de l'acide oxalique et du calcium en présence d'un agent chélateur ayant une affinité pour le calcium supérieure à celle de l'acide oxalique.

Selon une variante préférée, les plantes sont transformées avec une séquence d'ADN codant pour une protéine susceptible de dégrader l'acide oxalique associée à une séquence d'intérêt telle que définie précédemment, en particulier une séquence codant pour une protéine d'intérêt.

L'invention sera mieux comprise à l'aide de l'exposé ci-après, divisé en sections, qui comprend des résultats expérimentaux et une discussion de ceux-ci. Certaines de ces sections concement des expériences effectuées dans le but de réaliser l'invention, d'autres des exemples de réalisation de l'invention, donnés bien sûr à titre purement illustratif.

Dans cette partie expérimentale on utilise le clone gf-2.8 de la germine de blé décrite par B.G. Lane et al, 1991, J. Biol. Chem., 226- 10461-10469 ; la séquence de l'ADN génomique de ce clone est la séquence [SEQ ID N°16] et la séquence peptidique traduite est la séquence [SEQ ID N° 17].

Une grande partie de l'ensemble des techniques ci-après, bien connues de l'homme de l'art, est exposée en détail dans les ouvrages de Sambrook et al. : "Molecular Cloning : a Laboratory Manual", publié en 1989 par les éditions Cold Spring Harbor Press à New-York (2ème édition), et dans l'ouvrage de Gelvin et al: "Plant Molecular Biology Manual", publié en 1988 par les éditions Kluwer Academics.

### SECTION 1 : Purification et caractérisation partielle de l'oxalate oxydase d'orge

### 1) Purification de l'oxalate oxydase d'orge

Une oxalate oxydase d'orge a été purifiée jusqu'à homogénéité à partir d'une préparation commerciale enrichie en activité oxalate oxydase (Boehringer, ref 567 698) préparée à partir de grains d'orge en germination. La protéine est purifiée selon le protocole décrit ci-après :

### Etape 1:

La préparation commerciale lyophilisée est solubilisée dans l'eau puis équilibrée dans un tampon acétate 10mM de pH 5,2 par passage dans une mini-colonne à base de Sephadex G25 prête à l'emploi (NAP 10-Pharmacia). Cet extrait est fractionné par chromatographie sur une colonne d'échange d'ions à base de polymère synthétique (colonne Mono S HR5/5 de Pharmacia). Après dépôt de l'échantillon, les protéines non retenues sont éluées par le tampon acétate de sodium 10mM de pH 5,2. Les protéines retenues sur la colonne sont éluées par un gradient linéaire de 10 à 500mM de tampon acétate de sodium de pH 5,2.

L'éluat est analysé en ligne par son absorbance à 280 nm et les fractions collectées sont caractérisées : teneur en protéines mesurée par la technique colorimétrique de Bradford (1976, Anal. Biochem., 72, 248-252), activité oxalate oxydase mesurée selon la technique de Suguira et al., 1979, Chem. Pharm. Bull., 79, 2003-2007, décrite au point 2) ci-après. Chaque fraction est caractérisée, après électrophorèse en conditions dénaturantes (SDS) et coloration à l'argent, par sa mobilité électrophorétique comparée à des protéines de référence.

### Etape 2:

Les fractions présentant une activité oxalate oxydase et éluées à une concentration d'acétate de sodium comprise entre 200mM et 275mM sont rassemblées puis concentrées par centrifugation sur un système Centricon-10 (Amicon-réf. 4205). L'extrait est ensuite fractionné par chromatographie d'exclusion sur une colonne Superdex 75 (Pharmacia). Les fractions collectées sont analysées selon les méthodes décrites à l'étape 1.

A l'issue de la purification, on obtient une protéine unique, qui présente une activité oxalate oxydase, d'un poids moléculaire apparent de 26 ± 3 kDa (poids moléculaire déterminé après électrophorèse sur gel de polyacrylamide à 15 % en présence de SDS et révélation à l'argent).

### 2) Mesure de l'activité oxalate oxydase

L'activité oxalate oxydase est mesurée d'après la méthode décrite par Suguira et al. ,1979, Chem. Pharm. Bull. , 79, 2003-2007, et résumée ci-après. L'extrait enzymatique est incubé en présence de 75 µl d'oxalate de sodium (à 0,18 % dans du tampon succinate 100mM de pH 4,0) et de tampon succinate 100mM de pH 4,0, en quantité suffisante pour avoir un volume de mélange réactionnel égal à 1,5 ml.

Après 10 minutes d'incubation du mélange réactionnel à 37°C, on ajoute successivement 100 µl de tampon Tris 1 M de pH 8,9, puis 1 ml de réactif préparé extemporanément et constitué de : 8 mg de 4-aminoantipyrine, 6 mg de peroxydase de raifort (Sigma, ref P8250), 80 µl de diméthylaniline préparés dans 100 ml de tampon phosphate 0.1 M de pH 7,0.

L'activité enzymatique est estimée par la mesure spectrophotométrique de l'absorbance à 530 nm. Elle est exprimée en unité d'oxalate oxydase/mg de protéine (Une unité d'oxalate oxydase (U oxox) est la quantité d'enzyme qui convertit 1 µmole d'oxalate en peroxyde d'hydrogène en 1 minute à 37°C et à pH 3,8)

### 3) Caractérisation de la protéine purifiée

### a) Préparation d'anticorps polyclonaux

25 µg de la protéine d'orge purifiée jusqu'à homogénéité et ayant une activité oxalate oxydase sont injectés à un lapin dans 500 µl d'adjuvant complet de Freund (Sigma, ref F5881)

Trois injections de rappel de 25 µg dans l'adjuvant incomplet de Freund (500 µl) (Sigma, ref F5506) ont été réalisées à 3 semaines d'intervalle. L'immunsérum a été prélevé 3 semaines après la dernière injection.

Cet immunsérum reconnaît spécifiquement l'oxalate oxydase. Il permet de révéler cette protéine par la technique de Western blot (décrite dans la Section 2 4) b) à partir d'un extrait de protéines totales d'embryons d'orge en germination.

### b) Détermination de la séquence partielle de l'oxalate oxydase

Un échantillon de la protéine de 26 ± 3kDa ayant une activité oxalate oxydase est traité au bromure de cyanogène et les otigopeptides libérés sont séparés par HPLC phase inverse sur une colonne C4 Brownlee. La séquence N-terminale de la protéine ainsi que celle d'un peptide interne sont déterminées grâce à un séquenceur de protéines (Modèle 470A, Applied Biosystems, USA) équipé d'un chromatographe (Modèle 120A, Applied Biosystems) qui analyse en continu les dérivés phénylthiohydantoïques formés après chaque cycle de dégradation.

La séquence aminoterminale déterminée est la [SEC ID N° 18] suivante : Xaa étant un acide aminé non déterminé.

La séquence d'un peptide interne est la séquence [SEQ ID N° 19] suivante:

Après comparaison avec la banque des séquences protéiques connues (banque Swiss-Prot) en utilisant l'option GAP du logiciel UWGCG de l'Université du Wisconsin : Devereux et al., 1984, Nucl. Acids Res., 12, 387-395, une homologie d'au moins 94 % est trouvée avec la séquence d'une protéine de blé induite au cours de la germination, la germine, décrite par Lane et al, 1991 J. Biol. Chem., 226, 10461-10469.

### SECTION 2 : Transformation du tabac par le gène de la germine de blé, sélection sur acide oxalique des cals et des plantes transgéniques

### 1) Construction d'un vecteur de transformation

### a) Préparation de la séquence codant pour la germine de blé

Le fragment d'ADN HindIII - Sphl de 745 paires de bases du clone gf-2.8 décrit par B.G. Lane et al, (1991, J. Biol. Chem., 226, 10461-10469) portant la séquence codant pour la germine de blé a été purifié par électrophorèse sur gel d'agarose suivie par une extraction au moyen du kit "Geneclean" (Bio 101, ref 3105) selon le protocole du fabricant. Ce fragment comporte 19 paires de bases en amont de l'ATG initiateur ainsi que 54 paires de bases en aval du codon stop. Ce fragment a été inséré à l'aide de l'ADN ligase T4 entre les sites HindIII et Sphl du site de clonage multiple d'un vecteur pTZ19R (commercialisé par Pharmacia), dont le site BamHI a été détruit par remplissage grâce à la polymérase de Klenow, selon les méthodes bien connues de l'homme de l'art. Le plasmide ainsi créé est appelé plasmide pPH096. Le site HindIII présent dans ce plasmide est ensuite ouvert, et un nouveau site BamHI recréé par l'adjonction d'un oligonucléotide de séquence suivante [SEQ ID N°20] : AGCTGGATCC

Le vecteur obtenu, appelé plasmide pPH098, est cloné dans la souche *E. coli* JM 109 (Clontech). Après vérification de la séquence nucléotidique du fragment cloné, la partie codante est repurifiée sous la forme du fragment de restriction BamHI-SacI de 789 paires de bases. Ce fragment contient la séquence codant pour le peptide signal, ainsi que celle codant pour la germine mature tels qu'ils sont décrits par B. Lane et al, 1991, J. Biol. Chem., 226, 10461-10469.

### b) Préparation de la séquence promotrice comprenant le promoteur 35S du virus de la mosaïque du chou-fleur

A partir du plasmide pBI121 (Clontech) par coupure à l'aide des endonucléases HindIII et BamHI, puis électrophorèse sur gel d'agarose, le fragment HindIII-BamHI d'environ 900 paires de bases, contenant le promoteur 35S du virus de la mosaïque de chou-fleur, est isolé. Ce fragment est recoupé par HindIII. Le fragment d'environ 410 paires de bases, portant le site BamHI, est traité par l'ADN ligase T4 en présence d'un linker HindIII (séquence synthétique contenant un site HindIII). Après coupure par l'endonucléase HindIII et électrophorèse sur gel d'agarose, le fragment HindIII-BamHI résultant, d'environ 420 paires de bases, est isolé et purifié.

### c) Préparation de la séquence terminatrice comprenant le terminateur du gène de la nopaline synthase (NOS) d'Agrobacterium tumefaciens

A partir du plasmide pBI121 (Clontech), par coupure à l'aide des enzymes de restriction SacI et EcoRI, puis électrophorèse sur gel d'agarose, un fragment de 250 paires de bases environ, renfermant le terminateur du gène de la nopaline synthase, a été isolé.

### d) Clonage dans le vecteur binaire pBIN19

On a ligué à l'aide de l'ADN ligase T4 la séquence promotrice (cf. ci-dessus 1) b)), la séquence codant pour la germine (cf. ci-dessus 1) a)) et la séquence terminatrice (cf. ci-dessus 1) c)), dans le vecteur binaire pBIN19 (Bevan, 1984, Nucl. Acid Res., 12, 8711-8721), ouvert à l'aide des endonucléases HindIII et EcoRI. Ce vecteur porte deux gènes de résistance à la kanamycine, l'un pouvant s'exprimer dans les bactéries, l'autre situé immédiatement en amont du gène recombinant complet pouvant être transféré aux cellules végétales. Ce gène de résistance à la kanamycine servira à vérifier que les plantules régénérées obtenues après sélection sur acide oxalique à l'aide du gène codant pour l'oxalate oxydase sont effectivement transformées.

Le vecteur obtenu, appelé pPH100, est cloné dans la souche *E. coli* HB 101 (Clontech).

### 2) Transformation d'Agrobacterium tumefaciens

La transformation est réalisée selon la méthode de congélation-décongélation décrite dans Plant Molecular Biology Manual (Gelvin et al, op. cité) et résumée ci-après.

Des cellules compétentes d'*Agrobacterium tumefaciens* (souche LBA 4404, Clontech) sont préparées par refroidissement rapide dans la glace d'une culture en phase exponentielle de croissance. Les bactéries sont alors remises en suspension dans une solution de CaCl₂ 20mM. Des parties aliquotes de cette suspension sont distribuées dans des tubes Eppendorf, puis congelées dans l'azote liquide.

1 µg de plasmide pPH100 est ajouté aux cellules congelées, contenues dans un tube Eppendorf. La suspension est ensuite incubée à 37°C pendant 5 min ; 1 ml de milieu Luria (Gibco) est alors rajouté et le tube est incubé à 28°C pendant 4 h. Des parties aliquotes sont étalées sur des boîtes de Pétri contenant un milieu minimum gélosé, décrit dans Plant Molecular Biology Manual (op. cité), en présence de 100 mg de rifampicine et 25 mg/l de kanamycine. Dans ces conditions, seules poussent les colonies d'*Agrobacterium tumefaciens* ayant intégré le plasmide pPH100. Celles-ci contiennent le gène chimérique dans un contexte permettant sa réplication.

La résistance aux deux antibiotiques des colonies sélectionnées est vérifiée en repiquant celles-ci sur le même milieu de sélection deux fois de suite. La présence du gène chimérique associant le promoteur 35S à la partie codante de la germine de blé dans *Agrobacterium tumefaciens* est vérifiée par la méthode de Southern Blot sur une préparation d'ADN total (lyse des cellules, purification de l'ADN par extraction à l'aide du mélange phénol/chloroforme, selon le protocole décrit par Gelvin dans l'ouvrage cité ci-dessus, coupure de l'ADN purifié à l'aide d'enzymes de restriction, électrophorèse sur gel d'agarose, transfert sur membrane et hybridation, selon les techniques bien connues de l'homme de l'art).

### 3)Transformation du tabac

Du tabac *Nicotiana tabacum* cultivé in vitro a été infecté par *Aarobacterium tumefaciens* contenant le plasmide pPH100 selon la procédure de Horsch et al., bien connue de l'homme du métier (Horsch R.B. et al., 1985 Science 227, 1229-1231), dont les principales étapes sont exposées ci-après.

Des disques de feuilles de plantes axéniques de tabac *Nicotiana tabacum* (variété Wisconsin Havana 38) sont incubés dans une culture d'*A. tumefaciens* hébergeant le plasmide pPH100. Les disques égouttés sur papier Whatman sont mis en culture sur des milieux de culture en boîtes de Pétri afin de multiplier les cellules transformées de façon à obtenir des cals. Ces cals sont ensuite transférés sur du milieu contenant de la céfotaxime à 500 µg/ml destinée à décontaminer les tissus végétaux (élimination des *Agrobacterium tumefaciens*) et de la kanamycine à 100 µg/ml pour sélectionner le matériel transgénique. Des pousses transformées se développent à partir de ces cals ; les plantes qui en sont issues sont transférées en serres.

### 4) Mise en évidence de l'expression du gène de la germine dans le tabac transgénique

### a) Préparation des extraits de protéines de tabacs transformés et de tabacs témoins

Les fragments de tissus (cals et feuilles de plantes) ont été congelés dans l'azote liquide, réduits en poudre et stockés à -20°C.

Pour la réalisation d'électrophorèses, l'oxalate oxydase est extraite directement à partir de la poudre végétale par le tampon de charge de Laemmli (référence ci-dessous).

Pour les dosages d'activité oxalate oxydase, l'extrait enzymatique est réalisé par mise en suspension de la poudre végétale dans un tampon succinate 0,05M, pH 4.

Pour les dosages de protéines, l'extrait végétal, en suspension dans le tampon succinate ci-dessus, est centrifugé à 10 000 g pendant 5 min.

La concentration des protéines totales est déterminée sur les surnageants, appelés ci-après les extraits bruts de protéines, en suivant la technique de Bradford (Bradford M.M., 1976, Anal. Biochem., 72, 248-254).

### b) Immunodétection de l'oxalate oxydase (Westem blot) dans des cals et dans des plantes

On soumet les extraits bruts de protéines à un Western blot, technique bien connue de l'homme de l'art et décrite par H. Towbin et al., Proc. Ntl. Acad. Sci. USA, 76, 1979, 4350-4354, qui comprend les étapes suivantes:
· dénaturation par chauffage à 100°C pendant 10 min dans un tampon, dénommé tampon de charge, constitué de Tris 0,125 M, pH 6,8, SDS 4 %, bleu de bromophénol 0,002 %, glycérol 20 %, β-mercaptoéthanol 10 % (selon le protocole décrit par Laemmli U. K., 1970, Nature, 227, 680-685), suivie d'une centrifugation à 10 000 g ;
· séparation électrophorétique des différentes protéines contenues dans le solubilisat selon le protocole décrit par Laemmli (réf. ci-dessus) ;
· électrotransfert desdites protéines contenues dans le gel sur une membrane en PVDF (selon la technique de H. Towbin et al., 1979, Proc. Natl. Acad. Sci. USA 4350-4354).

L'immunodétection est réalisée selon un protocole qui comprend les étapes suivantes:
· saturation de la membrane PVDF(fluorure de polyvinylidène) sur laquelle les protéines ont été transférées par incubation pendant au minimum 2 h à 37°C dans une solution de gélatine à 3 % dans du tampon phosphate salin contenant 0,05 % de détergent Tween 20.
· incubation (pendant 1 h à 37°C) en présence de l'immunsérum préparé précédemment (contenant les anticorps polyclonaux reconnaissant la protéine recombinante), dilué au 1/10 000 dans du tampon phosphate salin.
· 3 lavages dans du tampon phosphate salin contenant 0,05 % de détergent Tween 20.

Le complexe antigène-anticorps est ensuite révélé à l'aide d'un système streptavidine-biotine conjugué à la phosphatase alcaline avec le kit RPN 23 d'Amersham ("Blotting detection kit"), utilisé selon les indications du fabricant.

L'empreinte obtenue montre, pour les cals et pour les feuilles de plantes de tabac transformées par le plasmide pPH100, la présence d'une protéine de poids moléculaire apparent d'environ 26 ± 3kDa, reconnue par les anticorps polyclonaux préparés dans la section 1 3)a) et absente des cals et des feuilles de plantes de tabac témoins. Cette protéine a le même poids moléculaire apparent que l'oxalate oxydase purifiée obtenue dans la Section 1.

### c) Mise en évidence de l'activité oxalate oxydase de la germine de blé exprimée dans du tabac

L'activité oxalate oxydase de 6 extraits de cals et de feuilles de plantes de tabac transformées par le plasmide pPH100 est mesurée selon la méthode de Suguira et al., décrite Section 1. Les résultats sont regroupés dans le Tableau I ci-après:

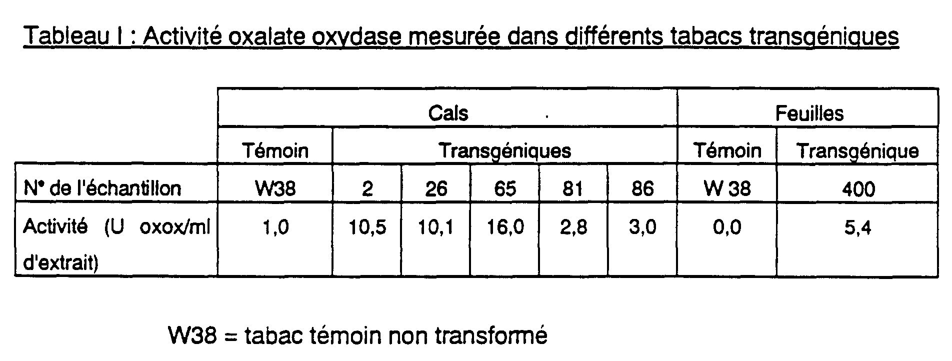

On constate à la lecture du tableau ci-dessus que le tabac transgénique (cals ou feuilles) présente une activité oxalate oxydase significativement supérieure à celle du tabac témoin.

### 5) Sélection sur acide oxalique des régénérants

### a) Protocole de sélection

Des disques de feuilles de plantes axéniques de tabac *Nicotiana tabacum* (variété Wisconsin Havana 38) sont incubés dans une culture d'*A. tumefaciens* hébergeant le plasmide pPH100. Les disques égouttés sur papier Whatman sont mis en culture sur des milieux de culture en boîtes de Pétri afin de multiplier les cellules transformées de façon à obtenir des cals. Après trois jours, les disques de feuilles sont rincés dans de l'éthanol 80 % puis dans du milieu gélosé de Murashige et Skoog (1962, Physiol. Plant. 15, 473) contenant 500 µg/ml de cefotaxime. Ils sont ensuite transférés sur un milieu contenant de l'acide oxalique afin de sélectionner les amas cellulaires exprimant l'oxalate oxydase.

### b) Mode de préparation des milieux de culture

L'acide oxalique s'associant au calcium pour former un sel d'oxalate de calcium insoluble, le milieu de sélection doit être préparé selon un protocole permettant de conserver le calcium sous une forme soluble utilisable par les cellules végétales malgré la présence d'acide oxalique.

### Préparation de la solution A :

Pour chaque concentration, 50 ml d'une solution d'acide oxalique concentrée 20 fois par rapport à la concentration finale attendue, sont ajustés à pH 5,8 avec une solution d'hydroxyde de potassium (KOH) 3M. La solution est ajustée à 100 ml avec de l'eau désionisée, stérilisée par filtration sur filtre de 0,45 µm, puis maintenue à une température de 50°C jusqu'à son utilisation.

### Préparation de la solution B :

258 mg d'EGTA (acide éthylène glycol-bis(β-amino ethyl ether) N,N,N',N'-tetraacétique) (Sigma, ref. E. 4378) sont mis en solution dans 100 ml d'eau désionisée. L'EGTA est solubilisé en ajustant le pH à 10,0 avec une solution de KOH 10 M. 100 mg de CaCl₂, 2H₂0 sont ensuite ajoutés et le pH de la solution est ajusté à 5,8 avec une solution HCl 2M. Le volume est ajusté à 150 ml puis la solution est stérilisée par passage sur filtre de 0,45 µm.

### Incorporation du calcium et de l'oxalate au milieu de culture :

750 ml de milieu de culture gélosé de Murashige et Skoog (1962, Physiol. Plant. 15, 473) sans chlorure de calcium, concentré 1,33 fois, sont autoclavés pendant 20 min à 120°C. La température est ensuite abaissée à 50°C et les solutions A et B incorporées dans ce milieu. Après homogénéisation de la solution, le milieu est coulé dans des boîtes de Pétri.

### c) Détermination de la dose d'acide oxalique à utiliser pour la sélection

Une gamme de sensibilité à l'acide oxalique de cals de tabac non transgénique (variété Wisconsin Havana 38) a été établie. Les milieux de culture ont été préparés selon le protocole décrit ci-dessus. Pour chaque concentration d'acide oxalique, 4 boîtes contenant 25 cals de diamètre de 2 mm et d'un poids moyen de 10 mg ont été mis en culture. Les résultats, exprimés en % d'inhibition de croissance pondérale par rapport aux cals témoins, sont présentés dans le tableau II ci-après:

**Tableau II :**

| Inhibition de la croissance pondérale de cals de tabac par l'acide oxalique | | | | | | | |
|---|---|---|---|---|---|---|---|
| Conc. Ac oxalique (µg/ml) | 0 | 40 | 60 | 80 | 120 | 180 | 270 |
| Inhib. croissance pondérale | 0 % | 0 % | 12 % | 19 % | 51 % | 89 % | 99 % |

Compte tenu des résultats ci-dessus, la dose de sélection choisie est de 270 µg/ml (3mM).

### d) Utilisation du gène codant pour l'oxalate oxydase comme gène de sélection des transformants

Après transformation et induction de la callogénèse, les disques foliaires de tabac sont transférés sur un milieu contenant 270 µg/ml d'acide oxalique. A cette concentration, les cals exprimant l'oxalate oxydase ne présentent pas une croissance différente du témoin poussant sur un milieu dépourvu d'acide oxalique et sont capables de survivre et de produire des plantes transgéniques. Le fait que les plantules régénérées sont bien transgéniques est vérifié par leur résistance à la kanamycine (second gène de sélection porté par le plasmide pPH100). Sur 29 plantes sélectionnées sur acide oxalique, 28 sont également résistantes à la kanamycine.

La sélection sur l'acide oxalique a donc permis de sélectionner les cals transformants ainsi que les plantes transgéniques.

### SECTION 3 : Transformation du tournesol par le gène de la germine de blé, sélection sur acide oxalique des cals

### 1)Transformation du tournesol

### Obtention de cals de tournesol transformés

Des graines de toumesol immatures sont prélevées sur le capitule de plantes de tournesol de la lignée HA89 bien connue, étudiée notamment par P.J. Goyne et al., Joumal Article n° 1534 of the North Dakota State Univ. Agric. Exp. Stn. Fargo ND-58105 et par M.F. Geriani, Plant Cell Physiol, 33, 2, 157-164. Ces graines sont stérilisées en surface pendant 30 min dans une solution d'hypochlorite de calcium à 2 %, puis rincées à l'eau distillée stérile.

Les embryons immatures sont prélevés sur ces graines et mis en culture sur le milieu I (tableau III) pendant 14 jours à 25°C et à l'obscurité. Ces embryons sont ensuite cultivés pendant 10 jours sur le milieu II à 25°C sous une photopériode de 16 h jour / 8 h nuit.

Les embryons sont alors coupés en deux au niveau de l'axe embryonnaire et mis à tremper pendant 10 min dans une suspension d'*Agrobacterium tumefaciens* contenant le vecteur binaire pPH100 (cf. sections 2.1 et 2.2). Cette suspension est obtenue par culture de cette bactérie pendant 15 h dans le milieu Luria liquide.

Les embryons sont ensuite égouttés sur du papier filtre stérile, puis remis en culture sur le milieu II à l'obscurité pendant 3 jours. Les embryons sont alors brièvement rincés par du milieu Murashige et Skoog liquide (Murashige et Skoog, 1962, Physiol. Plant 15 : 473) contenant 500 mg/l de l'antibiotique cefotaxime. Ils sont ensuite égouttés sur du papier filtre stérile et mis en culture sur du milieu III contenant 250 mg/l de cefotaxime, 250 mg/l de carbenicilline et 50 mg/l de paromomycine. Cette culture s'effectue à 25°C sous une photopériode de 16 h jour /8 h nuit ; les tissus végétaux ainsi que les cals se développant à leur surface sont repiqués tous les 21 jours sur ce même milieu.

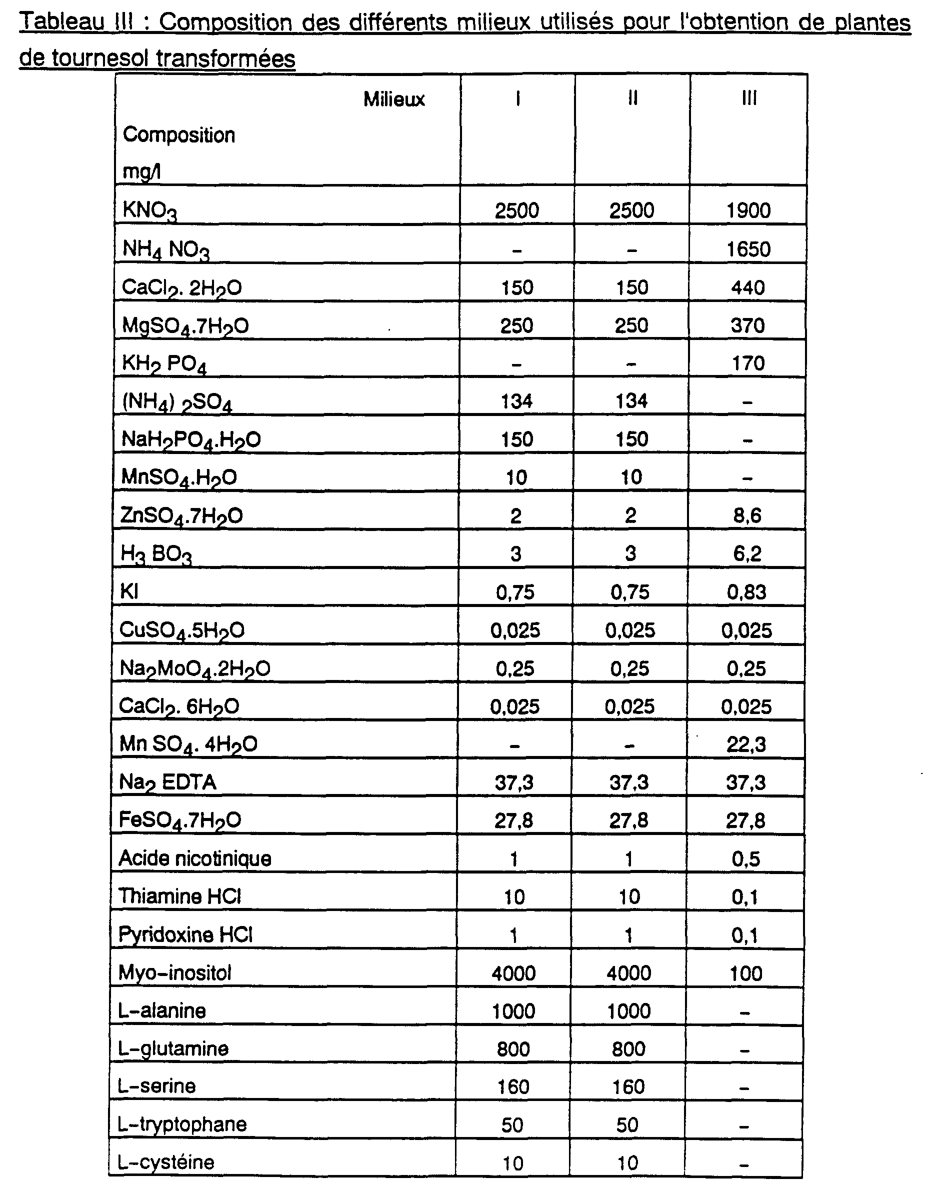

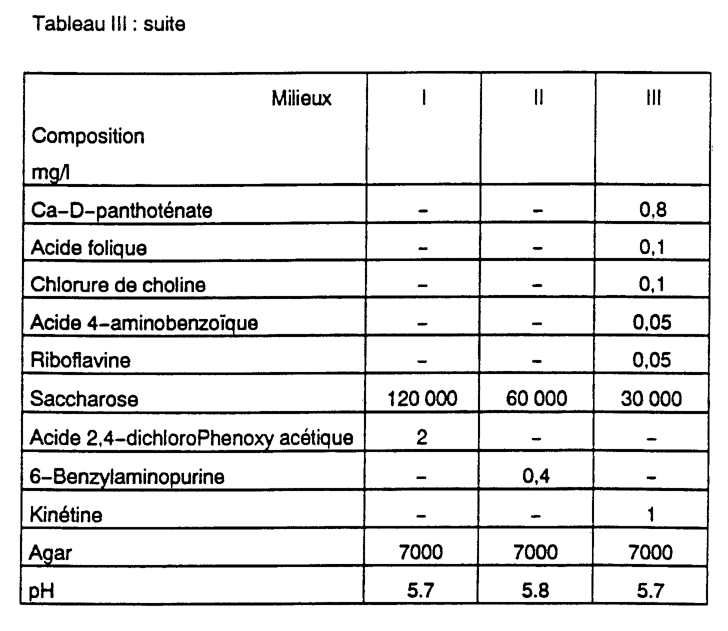

### 2) Mise en évidence de l'expression du gène de la germine dans le tournesol transgénique

### a) Préparation des extraits protéiques

Se fait d'une manière identique à celle décrite dans la Section 2 4)a).

### b) Immunodétection de l'oxalate oxydase (Westem blot) dans des cals

L'immunodétection, effectuée selon le protocole décrit dans la Section 2 4)b), met en évidence, dans des cals et des feuilles de tournesol transformé par le plasmide pPH100, la présence d'une protéine surnuméraire de poids moléculaire apparent d'environ 26 ± 3 kDa. Cette protéine, absente des cals et des feuilles de plantes témoins, a le même poids moléculaire apparent que l'oxalate oxydase purifiée obtenue dans la Section 1.

### c) Activité oxalate oxydase de la germine de blé exprimée dans du tournesol

L'activité oxalate oxydase de 5 extraits de protéines de cals de tournesol transformés par le plasmide pPH100 est mesurée selon la méthode de Suguira et al., décrite Section 1. Les résultats montrent une activité oxalate oxydase significativement supérieure à celle de l'extrait témoin.

**Tableau IV :**

| Activité oxalate oxydase de cals de tournesol transgéniques | | | | | | |
|---|---|---|---|---|---|---|
| N° du cal | Témoin non transformé | 17 | 18 | 19 | 20 | 21 |
| Unité Ox Ox/mg prot/min | 0,0 | 9,5 | 0,7 | 0,8 | 0,6 | 1,4 |

### 3)Sélection sur acide oxalique des cals transgéniques

La sélection des cals transgéniques est réalisée en cultivant le matériel végétal issu du milieu III (cf. ci-dessus 1) sur un milieux Murashige et Skoog de sélection préparé selon le protocole décrit dans la Section 2 5)b) .

La détermination de la dose de sélection à appliquer au cours de la culture de cals de tournesol se fait selon la méthode décrite dans la Section 2 5)c), cette méthode étant appliquée à des cals non transgéniques de tournesol obtenus à partir d'embryons immatures. Les résultats sont présentés dans le tableau V:

**Tableau V :**

| Inhibition de la croissance pondérale du tournesol par l'acide oxalique | | | | | | | | |
|---|---|---|---|---|---|---|---|---|
| Conc. Ac oxalique (µg/ml) | 0 | 40 | 70 | 90 | 140 | 180 | 235 | 270 |
| Inhib. croissance pondérale | 0 % | 0 % | 0 % | 0 % | 42 % | 92 % | 97 % | 99 % |

La dose d'acide oxalique permettant de réaliser la sélection est de 270 µg/ml (3mM). A cette concentration, les cals exprimant l'oxalate oxydase ne présentent pas d'inhibition de croissance.

### SECTION 4 : Utilisation de la sélection sur acide oxalique pour obtenir des plantes transgéniques exprimant un gène d'intérêt, codant, par exemple, pour une protéine à activité endochitinase.

### 1) Construction du vecteur de transformation

### a) Préparation du fragment portant le gène codant pour l'oxalate oxydase

Le fragment HindIII - EcoRI de 1420 pb environ du plasmide pPH100 décrit dans la section 2 d) est purifié et recloné dans un vecteur pUC19 selon les méthodes bien connues de l'homme de l'art. Ce plasmide est ensuite linéarisé grâce à l'endonucléase de restriction EcoRI et l'extrémité cohésive est remplie au moyen du fragment de Klenow. Puis, après coupure par l'endonucléase HindIII, le fragment HindIII - extrémité franche est purifié.

### b) Préparation du fragment portant un gène hybride codant pour une protéine à activité endochitinase

Le fragment HindIII - EcoRI provenant du plasmide pBR1 décrit dans la demande de brevet de WO 92/01792 exemple 1 et contenant un gène chimérique codant pour une protéine à activité endochitinase qui comprend le promoteur 35 S, une séquence codant pour une chitinase hybride tomate-tabac et le terminateur NOS est purifié, recloné dans le vecteur pUC19, puis le site HindIII est détruit d'une manière classique. Le fragment extrémité franche - EcoRI est purifié.

### c) Préparation d'un vecteur de transformation des plantes ne comportant pas de gène de résistance à la kanamycine

Le fragment NheI - HindIII comprenant la partie codant pour le gène de résistance à la kanamycine est éliminé du T-DNA du plasmide pBIN19 (cf Section 2 1 d). L'utilisation, selon les méthodes bien connues de l'homme de l'art, des oligonucléotides CTAGCA et AGCTTG permet de recirculariser le plasmide en recréant les sites de restriction Nhel et HindIII. Le plasmide résultant est ensuite linéarisé par les endonucléases de restriction HindIII et EcoRI.

### d) Assemblage du vecteur de transformation

On a ligué à l'aide de l'ADN ligase T4 le gène codant pour l'oxalate oxydase obtenu en a) ci-dessus) et le gène chimérique codant pour une protéine à activité chitinase (obtenu en b) ci-dessus) dans un vecteur binaire pBIN19 dans lequel on a éliminé le gène de résistance à la kanamycine s'exprimant dans les plantes (obtenu en c) ci-dessus).

Le vecteur obtenu, appelé pPH 106, est cloné dans la souche *E.Coli* HB 101 (Clontech)

### 2) Transformation d'Agrobacterium tumefaciens

La transformation est réalisée selon la méthode décrite dans la section 2 2).

### 3) Transformation du tabac

Du tabac *Nicotiana tabacum* cultivé in vitro est infecté par *Agrobacterium tumefaciens* contenant le plasmide pPH106 selon la procédure de Horsch et al., bien connue de l'homme du métier (Horsch R.B. et al., 1985 Science 227, 1229-1231), dont les principales étapes sont exposées ci-après.

Des disques de feuilles de plantes axéniques de tabac *Nicotiana tabacum* (variété Wisconsin Havana 38) sont incubés dans une culture d'*A. tumefaciens* hébergeant le plasmide pPH106. Les disques égouttés sur papier Whatman sont mis en culture sur des milieux de culture en boîtes de Pétri afin de multiplier les cellules transformées de façon à obtenir des cals. Après 48 heures, les disques sont rincés dans de l'éthanol 80 % puis dans du milieu gélosé de Murashige et Skoog (1962, Physiol. Plant. 15, 473) contenant 500 µg/ml de céfotaxime. Ils sont ensuite transférés pour 3 jours sur du milieu contenant de la céfotaxime à 500 µg/ml destinée à décontaminer les tissus végétaux (élimination des *Agrobacterium tumefaciens*).

### 4) Sélection sur acide oxalique des régénérants

Après transformation et induction de la callogénèse, les disques foliaires de tabac sont transférés sur un milieu contenant 270 µg/ml d'acide oxalique préparé selon la méthode décrite dans la section 2 5)b). Seuls les cals transgéniques exprimant le gène de l'oxalate oxydase, donc capables de dégrader l'acide oxalique, sont capables de survivre et de produire des plantes transgéniques.

### 5) Mise en évidence de l'expression de la protéine à activité endochitinase dans les tabacs transgéniques sélectionnés sur acide oxalique

### a) Préparation des extraits bruts de protéines de tabac transformé

Cette préparation est effectuée selon la méthode décrite dans la section 2 4)a)

### b) Mise en évidence de la chitinase hybride par immuno-empreinte (Western blot)

On soumet les extraits bruts de protéines à un Western blot, technique bien connue de l'homme de l'art et décrite par H. Towbin et al., Proc. Ntl. Acad. Sci. USA, 76, 1979, 4350-4354, qui comprend notamment les étapes mentionnées dans la section 2.4)b)

L'immunodétection de la protéine d'intérêt se réalise grâce à un immunsérum contenant des anticorps polyclonaux reconnaissant la protéine hybride à activité chitinase (cf. WO 92/01792 exemple 5).

Le complexe antigène-anticorps est ensuite révélé à l'aide d'un système streptavidine-biotine conjugué à la phosphatase alcaline avec le kit RPN 23 d'Amersham ("Blotting detection kit"), utilisé selon les indications du fabricant.

L'empreinte obtenue montre, pour les feuilles de plantes de tabac transformées par le plasmide pPH106, la présence d'une protéine de poids moléculaire apparent d'environ 26 ± 6 kDa reconnue par les anticorps polyclonaux et absente des feuilles des plantes de tabac témoins. Cette protéine a le même poids moléculaire apparent que la protéine hybride à activité chitinase décrite dans la demande WO 92/01792.

### c) Mise en évidence de l'activité chitinolytique de la protéine recombinante

L'activité chitinolytique des extraits bruts de protéines de feuilles de 5 plantes de tabac transformé par le plasmide pPH 106 (plantes n° 463, 464, 465, 468 et 469) et d'extrait brut de protéines de feuilles de plantes de tabac non transformé (plante W 38) est mesurée selon la méthode suivante:

L'activité endochitinase de la protéine est mesurée par une méthode radiochimique permettant d'estimer la quantité de monomères ou d'oligomères libérés par l'enzyme à partir d'un substrat (la chitine tritiée). Cette méthode, décrite par Molano et al. (1977, Anal. Biochem., 83, 648-656), est résumée ci-après.

A un volume d'extrait protéique de 10 µl sont ajoutés 50 µl d'une suspension de chitine tritiée d'activité spécifique 0,58 MBq/ml. Le volume final est ajusté à 300 µl avec du tampon d'acétate de sodium 0,2 M de pH 5,0. Après 90 min d'incubation à 30°C, la réaction d'hydrolyse de la chitine est arrêtée par 100 µl d'acide trichloracétique à 20 %. Les tubes réactionnels sont ensuite centrifugés pendant 10 min à 12 000 g. Une partie aliquote de 100 µl du sumageant renfermant les oligomères solubles de chitine est prélevée et la radioactivité correspondante est mesurée par scintillation liquide en présence de 5 ml de mélange scintillant. On exprime l'activité chitinolytique spécifique en dpm/µg de protéine.

Pour les 5 plantes sélectionnées sur acide oxalique, on obtient les valeurs suivantes:

| | | | | | | |
|---|---|---|---|---|---|---|
| genotype | W 38 | 463 | 464 | 465 | 468 | 469 |
| Activité spécifique DPM/µg prot | 95 | 149 | 348 | 318 | 301 | 320 |
| (W 38 = tabac témoin non transformé) | | | | | | |

On constate sur le tableau ci-dessus que les extraits de plantes de tabac transformé par le plasmide pPH106 ont une activité chitinolytique significativement supérieure à celle de l'extrait de plantes de tabac-témoin. La sélection sur acide oxalique permet donc d'obtenir des plantes exprimant un gène d'intérêt, en l'occurence le gène hybride codant pour une protéine à activité chitinase décrit dans la demande de brevet WO 92/01792.

## Revendications

1. Utilisation d'une séquence d'ADN codant pour une protéine susceptible de dégrader l'acide oxalique à titre d'agent de sélection de cellules végétales.

2. Utilisation selon la revendication 1, **caractérisée en ce que** ladite séquence d'ADN est associée à une séquence d'intérêt.

3. Utilisation selon l'une des revendications 1ou 2, **caractérisée en ce que** la protéine susceptible de dégrader l'acide oxalique est une enzyme à activité oxydase.

4. Utilisation selon la revendication 3, **caractérisée en ce que** l'enzyme à activité oxydase est l'oxalate oxydase de séquence [SEQ ID N° 1] ci-après ou une séquence présentant un degré d'homologie élevé avec la séquence [SEQ ID N° 1] ci-après :

5. Utilisation selon l'une des revendications 1 ou 2, **caractérisée en ce que** la séquence d'ADN codant pour une protéine susceptible de dégrader l'acide oxalique est la séquence [SEQ ID N°2] ci-après :

6. Utilisation selon l'une des revendications 1 ou 2, **caractérisée en ce que** la protéine susceptible de dégrader l'acide oxalique est une décarboxylase

7. Utilisation selon l'une des revendications 2 à 4, **caractérisée en ce que** la séquence d'intérêt code pour une protéine qui confère aux plantes une résistance aux agents pathogènes.

8. Utilisation selon la revendication 7, **caractérisée en ce que** la séquence d'intérêt code pour une protéine à activité endochitinase.

9. Procédé pour sélectionner sur acide oxalique des cals ou des plantes transformés par une séquence d'ADN codant pour une protéine susceptible de dégrader l'acide oxalique, **caractérisé en ce qu'**il consiste à cultiver les cals ou les plantes sur un milieu de culture contenant de l'acide oxalique et du calcium en présence d'un agent chélateur ayant une affinité pour le calcium supérieure à celle de l'acide oxalique.

10. Procédé selon la revendication 9, **caractérisé en ce que** ladite séquence d'ADN est associée à une séquence d'intérêt.

11. Procédé selon la revendication 9, **caractérisé en ce que** les agents chélateurs sont choisis parmi l'EDTA et l'EGTA.

12. Procédé selon l'une quelconque des revendications 9 à 11 **caractérisé en ce que** les cals ou les plantes appartiennent à l'une des espèces *Nicotiana tabacum, Helianthus annuus* et *Brassica napus.*

## Claims

1. Use of a DNA sequence coding for a protein capable of degrading oxalic acid as selection agent for plant cells.

2. Use according to Claim 1, **characterized in that** the said DNA sequence is associated with a sequence of interest.

3. Use according to one of Claims 1 and 2, **characterized in that** the protein capable of degrading oxalic acid is an enzyme with oxidase activity.

4. Use according to Claim 3, **characterized in that** the enzyme with oxidase activity is the oxalate oxidase of sequence [SEQ ID No.1] below or a sequence having a high degree of homology with the sequence [SEQ ID No.1] below:

5. Use according to one of Claims 1 and 2, **characterized in that** the DNA sequence coding for a protein capable of degrading oxalic acid is the sequence [SEQ ID No.2] below:

6. Use according to one of Claims 1 and 2, **characterized in that** the protein capable of degrading oxalic acid is a decarboxylase.

7. Use according to one of Claims 2 to 4, **characterized in that** the sequence of interest codes for a protein which confers a resistance to pathogenic agents to plants.

8. Use according to Claim 6, **characterized in that** the sequence of interest codes for a protein with endochitinase activity.

9. Process for selecting, on oxalic acid, calluses or transformed plants by a DNA sequence coding for a protein capable of degrading oxalic acid, **characterized in that** it consists of culturing the calluses or the plants on a culture medium containing oxalic acid and calcium in the presence of a chelating agent having an affinity for calcium higher than that of oxalic acid.

10. Process according to Claim 9, **characterized in that** the said DNA sequence is associated with a sequence of interest.

11. Process according to Claim 9, **characterized in that** the chelating agents are chosen from amongst EDTA and EGTA.

12. Process according to any one of Claims 9 to 11, **characterized in that** the calluses or the plants belong to one of the species *Nicotiana tabacum, Helianthus annuus* and *Brassica napus.*

## Patentansprüche

1. Verwendung einer DNA-Sequenz, die für ein Protein kodiert, das fähig ist, Oxalsäure als Selektionsmittel für Pflanzenzellen zu degradieren.

2. Verwendung gemäß Anspruch 1, **dadurch gekennzeichnet, daß** die DNA-Sequenz mit einer Sequenz von Interesse verbunden ist.

3. Verwendung gemäß einem der Ansprüche 1 oder 2, **dadurch gekennzeichnet, daß** das Protein, das fähig ist, Oxalsäure zu degradieren, ein Enzym mit Oxidase-Wirkung ist.

4. Verwendung gemäß Anspruch 3, **dadurch gekennzeichnet, daß** das Enzym mit Oxidase-Wirkung Oxalatoxidase mit der folgenden Sequenz (SEQ ID No.1) ist oder eine Sequenz, die einen erhöhten Grad der Homologie mit der folgenden Sequenz (SEQ ID No.1) aufweist:

5. Verwendung gemäß einem der Ansprüche 1 oder 2, **dadurch gekennzeichnet, daß** die DNA-Sequenz, die für ein Protein kodiert, das fähig ist, Oxalsäure zu degradieren, die folgende Sequenz (SEQ ID No.2) ist:

6. Verwendung gemäß einem der Ansprüche 1 oder 2, **dadurch gekennzeichnet, daß** das Protein, das fähig ist, Oxalsäure zu degradieren, eine Decarboxylase ist.

7. Verwendung gemäß einem der Ansprüche 2 bis 4, **dadurch gekennzeichnet, daß** die Sequenz von Interesse für ein Protein kodiert, das den Pflanzen eine Resistenz gegenüber Krankheitserregern vermittelt.

8. Verwendung gemäß Anspruch 7, **dadurch gekennzeichnet, daß** die Sequenz von Interesse für ein Protein mit Endochitinase-Aktivität kodiert.

9. Verfahren zum Selektionieren von Kallussen oder transformierten Pflanzen durch eine DNA-Sequenz, die für ein Protein kodiert, das fähig ist, Oxalsäure zu degradieren, **dadurch gekennzeichnet, daß** es darin besteht, die Kallusse oder die Pflanzen in einem Kulturmedium zu kultivieren, das Oxalsäure und Kalzium in Gegenwart eines Chelators mit einer Affinität für Kalzium, die über der der Oxalsäure liegt, enthält.

10. Verfahren gemäß Anspruch 9, **dadurch gekennzeichnet, daß** die DNA-Sequenz mit einer Sequenz von Interesse assoziiert ist.

11. Verfahren gemäß Anspruch 9, **dadurch gekennzeichnet, daß** die Chelatoren aus EDTA und EGTA ausgewählt sind.

12. Verfahren gemäß einem der Ansprüche 9 bis 11, **dadurch gekennzeichnet, daß** die Kallusse oder die Pflanzen einer der Arten Nicotiana tabacum, Helianthus annuus und Brassica napus angehören.


### LISTE DE SEQUENCES

(1) INFORMATION GENERALE:
   (i) DEPOSANT:
      (A) NOM: BIOGEMMA
      (B) RUE: 1 rue Edouard Colonne
      (C) VILLE: PARIS
      (E) PAYS: France
      (F) CODE POSTAL: 75001
      (G) TELEPHONE: 01.55.34.94.00
      (H) TELECOPIE: 01.55.34.94.01
   (ii) TITRE DE L' INVENTION: Utilisation d'une séquence d'ADN codant pour une protéine susceptible de dégrader l'acide oxalique à titre de gène de sélection
   (iii) NOMBRE DE SEQUENCES: 20
   (iv) FORME LISIBLE PAR ORDINATEUR:
      (A) TYPE DE SUPPORT: Floppy disk
      (B) ORDINATEUR: IBM PC compatible
      (C) SYSTEME D' EXPLOITATION: PC-DOS/MS-DOS
      (D) LOGICIEL: PatentIn Release #1.0, Version #1.25 (OEB)
   (vi) DONNEES DE LA DEMANDE ANTERIEURE:
      (A) NUMERO DE DEPOT: FR 92 14721
      (B) DATE DE DEPOT: 07-DEC-1992
(2) INFORMATION POUR LA SEQ ID NO: 1:
   (i) CARACTERISTIQUES DE LA SEQUENCE:
      (A) LONGUEUR: 224 acides aminés
      (B) TYPE: acide aminé
      (D) CONFIGURATION: linéaire
   (ii) TYPE DE MOLECULE: protéine
   (xi) DESCRIPTION DE LA SEQUENCE: SEQ ID NO: 1:
   Met Gly Tyr Ser Lys Thr Leu Val Ala Gly Leu Phe Ala Met Leu Leu
   1 5 10 15
   Leu Ala Pro Ala Val Leu Ala Thr Asp Pro Asp Pro Leu Gln Asp Phe
   20 25 30
   Cys Val Ala Asp Leu Asp Gly Lys Ala Val Ser Val Asn Gly His Thr
   35 40 45
   Cys Lys Pro Met Ser Glu Ala Gly Asp Asp Phe Leu Phe Ser Ser Lys
   50 55 60
   Leu Ala Lys Ala Gly Asn Thr Ser Thr Pro Asn Gly Ser Ala Val Thr
   65 70 75 80
   Glu Leu Asp Val Ala Glu Trp Pro Gly Thr Asn Thr Leu Gly Val Ser
   85 90 95
   Met Asn Arg Val Asp Phe Ala Pro Gly Gly Thr Asn Pro Pro His Ile
   100 105 110
   His Pro Arg Ala Thr Glu Ile Gly Ile Val Met Lys Gly Glu Leu Leu
   115 120 125
   Val Gly Ile Leu Gly Ser Leu Asp Ser Gly Asn Lys Leu Tyr Ser Arg
   130 135 140
   Val Val Arg Ala Gly Glu Thr Phe Leu Ile Pro Arg Gly Leu Met His
   145 150 155 160
   Phe Gln Phe Asn Val Gly Lys Thr Glu Ala Ser Met Val Val Ser Phe
   165 170 175
   Asn Ser Gln Asn Pro Gly Ile Val Phe Val Pro Leu Thr Leu Phe Gly
   180 185 190
   Ser Asn Pro Pro Ile Pro Thr Pro Val Leu Thr Lys Ala Leu Arg Val
   195 200 205
   Glu Ala Arg Val Val Glu Leu Leu Lys Ser Lys Phe Ala Ala Gly Phe
   210 215 220
(2) INFORMATION POUR LA SEQ ID NO: 2:
   (i) CARACTERISTIQUES DE LA SEQUENCE:
      (A) LONGUEUR: 672 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (xi) DESCRIPTION DE LA SEQUENCE: SEQ ID NO: 2:
   ATGGGGTACT CCAAAACCCT AGTAGCTGGC GTGTTCGCAA TGCTGTTACT AGCTCCGGCC 60
   GTCTTGGCCA CCGACCCAGA CCCTCTCCAG GACTTCTGTG TCGCCGACCT CGACGGCAAG 120
   GCGGTCTCGG TGAACGGGCA CACGTGCAAG CCCATGTCGG AGGCCGGCGA CGACTTCCTC 180
   TTCTCGTCCA AGTTGGCCAA GGCCGGCAAC ACGTCCACCC CGAACGGCTC CGCCGTGACG 240
   GAGCTCGACG TGGCCGAGTG GCCCGGTACC AACACGCTGG GTGTGTCCAT GAACCGCGTG 300
   GACTTTGCTC CCGGAGGCAC CAACCCACCA CACATCCACC CGCGTGCCAC CGAGATCGGC   360
   ATCGTGATGA AAGGTGAGCT TCTCGTGGGA ATCCTTGGCA GCCTCGACTC CGGGAACAAG   420
   CTCTACTCGA GGGTGGTGCG CGCCGGAGAG ACGTTCCTCA TCCCACGGGG CCTCATGCAC   480
   TTCCAGTTCA ACGTCGGTAA GACCGAGGCC TCCATGGTCG TCTCCTTCAA CAGCCAGAAC   540
   CCCGGCATTG TCTTCGTGCC CCTCACGCTC TTCGGCTCCA ACCCGCCCAT CCCAACGCCG   600
   GTGCTCACCA AGGCACTCCG GGTGGAGGCC AGGGTCGTGG AACTTCTCAA GTCCAAGTTT   660
   GCCGCTGGGT TT                                                       672
(2) INFORMATION POUR LA SEQ ID NO: 3:
   (i) CARACTERISTIQUES DE LA SEQUENCE:
      (A) LONGUEUR: 254 acides aminés
      (B) TYPE: acide aminé
      (D) CONFIGURATION: linéaire
   (ii) TYPE DE MOLECULE: protéine
   (xi) DESCRIPTION DE LA SEQUENCE: SEQ ID NO: 3:
   Gly Gly Asp Leu Gly Ser Val Ile Ser Asn Ser Met Phe Asp Gln Met
   1               5                   10                  15
   Leu Lys His Arg Asn Glu Asn Ser Cys Gln Gly Lys Asn Asn Phe Tyr
               20                  25                  30
   Ser Tyr Asn Ala Phe Ile Thr Ala Ala Arg Ser Phe Pro Gly Phe Gly
           35                  40                  45
   Thr Ser Gly Asp Ile Asn Ala Arg Lys Arg Glu Ile Ala Ala Phe Phe
       50                  55                  60
   Ala Gln Thr Ser His Glu Thr Thr Gly Gly Trp Pro Ser Ala Pro Asp
   65                  70                  75                  80
   Gly Pro Phe Ala Trp Gly Tyr Cys Phe Leu Arg Glu Arg Gly Asn Pro
                   85                  90                  95
   Gly Asp Tyr Cys Ser Pro Ser Ser Gln Trp Pro Cys Ala Pro Gly Arg
               100                 105                 110
   Lys Tyr Phe Gly Arg Gly Pro Ile Gln Ile Ser His Asn Tyr Asn Tyr
           115                 120                 125
   Gly Pro Cys Gly Arg Ala Ile Gly Val Asp Leu Leu Asn Asn Pro Asp
       130                 135                 140
   Leu Val Ala Thr Asp Pro Val Ile Ser Phe Lys Thr Ala Ile Trp Phe
   145                 150                 155                 160
   Trp Met Thr Pro Gln Ser Pro Lys Pro Ser Cys His Asp Val Ile Ile
                   165                 170                 175
   Gly Arg Trp Asn Pro Ser Ala Gly Asp Arg Ser Ala Asn Arg Leu Pro
               180                 185                 190
   Gly Phe Gly Val Ile Thr Asn Ile Ile Asn Gly Gly Leu Glu Cys Gly
           195                 200                 205
   Arg Gly Asn Asp Asn Arg Val Gln Asp Arg Ile Gly Phe Tyr Arg Arg
       210                 215                 220
   Tyr Cys Gly Ile Leu Gly Val Ser Pro Gly Asp Asn Leu Asp Cys Gly
   225                 230                 235                 240
   Asn Gln Arg Ser Phe Gly Asn Gly Leu Leu Val Asp Thr Met
                   245                 250
(2) INFORMATION POUR LA SEQ ID NO: 4:
   (i) CARACTERISTIQUES DE LA SEQUENCE:
      (A) LONGUEUR: 24 acides aminés
      (B) TYPE: acide aminé
      (D) CONFIGURATION: linéaire
   (ii) TYPE DE MOLECULE: peptide
   (xi) DESCRIPTION DE LA SEQUENCE: SEQ ID NO: 4:
   Met Arg Arg Thr Ser Lys Leu Thr Thr Phe Ser Leu Leu Phe Ser Leu
   1               5                   10                  15
   Val Leu Leu Ser Ala Ala Leu Ala
               20
(2) INFORMATION POUR LA SEQ ID NO: 5:
   (i) CARACTERISTIQUES DE LA SEQUENCE:
      (A) LONGUEUR: 51 acides aminés
      (B) TYPE: acide aminé
      (D) CONFIGURATION: linéaire
   (ii) TYPE DE MOLECULE: peptide
   (xi) DESCRIPTION DE LA SEQUENCE: SEQ ID NO: 5:
   Gln Asn Cys Gly Ser Gln Gly Gly Gly Lys Val Cys Ala Ser Gly Gln
   1               5                   10                  15
   Cys Cys Ser Lys Phe Gly Trp Cys Gly Asn Thr Asn Asp His Cys Gly
               20                  25                  30
   Ser Gly Asn Cys Gln Ser Gln Cys Pro Gly Gly Gly Pro Gly Pro Gly
           35                  40                  45
   Pro Val Thr
       50
(2) INFORMATION POUR LA SEQ ID NO: 6:
   (i) CARACTERISTIQUES DE LA SEQUENCE:
      (A) LONGUEUR: 1153 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (ix) CARACTERISTIQUE ADDITIONELLE:
      (A) NOM/CLE: intron
      (B) EMPLACEMENT: 443..521
   (ix) CARACTERISTIQUE ADDITIONELLE:
      (A) NOM/CLE: intron
      (B) EMPLACEMENT: 676..756
   (xi) DESCRIPTION DE LA SEQUENCE: SEQ ID NO: 6:
   ATGAGGCGAA CTTCTAAATT GACTACTTTT TCTTTGCTGT TTTCTCTGGT TTTGCTGAGT    60
   GCTGCCTTGG CACAGAATTG TGGTTCACAG GGCGGAGGCA AAGTTTGTGC GTCGGGACAA   120
   TGTTGCAGCA AATTCGGGTG GTGCGGTAAC ACTAATGACC ATTGTGGTTC TGGCAATTGT   180
   CAAAGTCAGT GTCCAGGTGG CGGCCCTGGT CCTGGTCCTG TTACTGGTGG GGACCTCGGA   240
   AGCGTCATCT CAAATTCTAT GTTTGATCAA ATGCTTAAGC ATCGTAACGA AAATTCTTGT   300
   CAAGGAAAGA ATAATTTCTA CAGTTACAAT GCCTTTATTA CTGCTGCTAG GTCTTTTCCT   360
   GGCTTTGGTA CAAGTGGTGA TATCAATGCC CGTAAAAGGG AAATTGCTGC TTTCTTTGCC   420
   CAAACCTCCC ATGAAACTAC TGGTATGTGT ATAACCATTC ACATCGAACC ATTAAAATAT    480
   AATTTCATTT TATTTTATTT AGTAATTGAT TATATATGTA GGAGGATGGC CTTCCGCACC    540
   TGATGGACCA TTCGCATGGG GTTACTGTTT CCTTAGAGAA CGAGGTAACC CCGGTGACTA    600
   CTGTTCACCA AGTAGTCAAT GGCCTTGTGC ACCTGGAAGG AAATATTTCG GACGAGGCCC    660
   AATCCAAATT TCACAGTAAG CTACATAAAT CTATATATGG TAAAATTTGA TGAACTTGTA    720
   GTGTCTAATT ACGTGTATTT TGACATTTCA AAACAGCAAC TACAACTATG GGCCATGTGG    780
   AAGAGCCATC GGAGTGGACC TTTTAAACAA TCCTGATTTA GTAGCCACAG ACCCAGTCAT    840
   CTCATTCAAG ACTGCTATCT GGTTCTGGAT GACCCCTCAA TCACCAAAGC CTTCTTGCCA    900
   CGATGTCATC ATTGGAAGAT GGAACCCATC TGCCGGTGAC CGATCAGCCA ATCGTCTTCC    960
   TGGATTTGGT GTCATCACAA ACATCATCAA TGGGGGCCTG GAATGTGGTC GTGGCAATGA   1020
   CAATAGGGTC CAGGATCGCA TTGGGTTTTA CAGGAGGTAT TGCGGTATTC TTGGTGTTAG   1080
   TCCTGGTGAC AATCTTGATT GCGGAAACCA GAGATCTTTT GGAAACGGAC TTTTAGTCGA   1140
   TACTATGTAA TGA                                                      1153
(2) INFORMATION POUR LA SEQ ID NO: 7:
   (i) CARACTERISTIQUES DE LA SEQUENCE:
      (A) LONGUEUR: 389 acides aminés
      (B) TYPE: acide aminé
      (D) CONFIGURATION: linéaire
   (ii) TYPE DE MOLECULE: protéine
   (xi) DESCRIPTION DE LA SEQUENCE: SEQ ID NO: 7:
   Gly Ser Gly Phe Ala Asn Ala Val Tyr Phe Thr Asn Trp Gly Ile Tyr
   1               5                   10                  15
   Gly Arg Asn Phe Gln Pro Ala Asp Leu Pro Ala Ser Glu Ile Thr His
               20                  25                  30
   Val Leu Tyr Ser Phe Met Asn Val Arg Ala Asp Gly Thr Ile Phe Ser
           35                  40                  45
   Gly Asp Thr Tyr Ala Asp Tyr Glu Lys His Tyr Ala Gly Asp Ser Trp
       50                  55                  60
   Asn Asp Val Gly Thr Asn Ala Tyr Gly Cys Val Lys Gln Leu Tyr Leu
   65                  70                  75                  80
   Leu Lys Lys Gln Asn Arg Asn Met Lys Val Met Leu Ser Ile Gly Gly
                   85                  90                  95
   Trp Thr Trp Ser Thr Asn Phe Pro Ala Ala Ala Ser Ser Ala Ala Thr
               100                 105                 110
   Arg Lys Thr Phe Ala Gln Ser Ala Val Gly Phe Met Lys Asp Trp Gly
           115                 120                 125
   Phe Asp Gly Ile Asp Ile Asp Trp Glu Tyr Pro Ala Asp Ala Thr Gln
       130                 135                 140
   Ala Gln Asn Met Val Leu Leu Leu Gln Ala Val Arg Ser Glu Leu Asp
   145                 150                 155                 160
   Ser Tyr Ala Ala Gln Tyr Ala Lys Gly His His Phe Leu Leu Ser Ile
                   165                 170                 175
   Ala Ala Pro Ala Gly Pro Asp Asn Tyr Asn Lys Leu Lys Phe Ala Glu
               180                 185                 190
   Leu Gly Lys Val Leu Asp Tyr Ile Asn Leu Met Ala Tyr Asp Tyr Ala
           195                 200                 205
   Gly Ser Trp Ser Asn Tyr Thr Gly His Asp Ala Asn Ile Tyr Ala Asn
   210 215 220
   Pro Gln Asn Pro Asn Ala Thr Pro Tyr Asn Thr Asp Asp Ala Val Gln
   225 230 235 240
   Ala Tyr Ile Asn Gly Gly Val Pro Ala Asn Lys Ile Val Leu Gly Met
   245 250 255
   Pro Ile Tyr Gly Arg Ser Phe Gln Gln Thr Glu Gly Ile Gly Lys Pro
   260 265 270
   Tyr Asn Gly Ile Gly Ser Gly Ser Trp Glu Asn Gly Ile Trp Asp Tyr
   275 280 285
   Lys Ala Leu Pro Lys Ala Gly Ala Thr Val Lys Cys Asp Asp Thr Ala
   290 295 300
   Lys Gly Cys Tyr Ser Tyr Asp Pro Ser Thr Lys Glu Leu Ile Ser Phe
   305 310 315 320
   Asp Thr Pro Ala Met Ile Ser Thr Lys Val Ser Trp Leu Lys Gly Lys
   325 330 335
   Gly Leu Gly Gly Ser Met Phe Trp Glu Ala Ser Ala Asp Lys Lys Gly
   340 345 350
   Ser Asp Ser Leu Ile Ser Thr Ser His Gln Gly Leu Gly Ser Gln Asp
   355 360 365
   Ser Thr Gln Asn Tyr Leu Asp Tyr Pro Asn Ser Lys Tyr Asp Asn Ile
   370 375 380
   Lys Lys Gly Met Asn
   385
(2) INFORMATION POUR LA SEQ ID NO: 8:
   (i) CARACTERISTIQUES DE LA SEQUENCE:
      (A) LONGUEUR: 22 acides aminés
      (B) TYPE: acide aminé
      (D) CONFIGURATION: linéaire
   (ii) TYPE DE MOLECULE: peptide
   (vii) SOURCE IMMEDIATE:
      (B) CLONE: signal peptide
   (xi) DESCRIPTION DE LA SEQUENCE: SEQ ID NO: 8:
   Met Leu Ser Phe Val Lys Lys Ser Ile Ala Leu Val Ala Ala Leu Gln
   1               5                   10                  15
   Ala Val Thr Ala Leu Ala
               20
(2) INFORMATION POUR LA SEQ ID NO: 9:
   (i) CARACTERISTIQUES DE LA SEQUENCE:
      (A) LONGUEUR: 12 acides aminés
      (B) TYPE: acide aminé
      (D) CONFIGURATION: linéaire
   (ii) TYPE DE MOLECULE: peptide
   (xi) DESCRIPTION DE LA SEQUENCE: SEQ ID NO: 9:
   Thr Pro Ile Ser Ser Glu Ala Gly Val Glu Lys Arg
   1               5                   10
(2) INFORMATION POUR LA SEQ ID NO: 10:
   (i) CARACTERISTIQUES DE LA SEQUENCE:
      (A) LONGUEUR: 1167 paires de bases
      (B) TYPE: acide nucléique
      (C) NOMBRE DE BRINS: simple
      (D) CONFIGURATION: linéaire
   (ii) TYPE DE MOLECULE: ADNc
   (xi) DESCRIPTION DE LA SEQUENCE: SEQ ID NO: 10:
   GGTAGTGGTT TTGCAAATGC CGTCTACTTC ACCAACTGGG GCATTTATGG CCGCAACTTC 60
   CAGCCTGCCG ACCTTCCTGC CTCGGAGATT ACTCACGTAC TCTACTCCTT CATGAATGTC 120
   CGCGCAGATG GCACCATCTT TTCCGGTGAT ACCTATGCCG ACTACGAGAA GCACTACGCT 180
   GGTGACTCTT GGAACGATGT GGGCACGAAC GCTTACGGTT GTGTTAAGCA ACTTTATCTT 240
   CTCAAGAAGC AGAACCGCAA CATGAAGGTG ATGCTGTCGA TTGGTGGTTG GACATGGTCT 300
   ACCAACTTCC CCGCTGCCGC CAGCTCGGCT GCTACCCGAA AGACTTTTGC TCAGTCTGCT 360
   GTTGGCTTCA TGAAGGACTG GGGTTTCGAC GGTATTGATA TCGACTGGGA GTACCCCGCC 420
   GATGCCACTC AGGCTCAGAA TATGGTTCTC TTGCTACAGG CTGTCCGCAG TGAGCTCGAC 480
   TCCTACGCTG CCCAGTACGC CAAGGGTCAC CACTTCCTGC TTTCAATTGC CGCCCCTGCT 540
   GGACCTGACA ATTATAACAA GCTGAAGTTT GCTGAGCTTG GCAAGGTTCT CGATTACATT 600
   AACCTCATGG CTTACGATTA CGCTGGATCT TGGAGCAACT ACACTGGCCA CGATGCCAAC 660
   ATATACGCAA ACCCGCAGAA CCCCAACGCC ACCCCTTACA ACACGGACGA TGCTGTCCAG 720
   GCCTATATCA ACGGCGGCGT CCCTGCCAAC AAGATCGTCC TTGGTATGCC AATCTACGGC 780
   CGATCCTTCC AGCAAACCGA GGGTATCGGT AAGCCTTACA ATGGTATTGG CTCTGGTAGC 840
   TGGGAGAACG GTATCTGGGA CTACAAGGCT CTCCCCAAGG CTGGTGCCAC CGTCAAGTGC 900
   GACGATACCG CCAAGGGATG CTACAGCTAC GATCCAAGCA CTAAGGAGCT TATTTCTTTC 960
   GATACGCCGG CTATGATCAG CACCAAAGTT AGCTGGCTCA AGGGCAAGGG CCTTGGCGGC 1020
   AGCATGTTCT GGGAGGCTTC TGCCGACAAG AAGGGCTCGG ACTCTCTTAT TAGCACCAGC 1080
   CACCAAGGTC TCGGTAGCCA GGACAGCACT CAGAACTACC TCGACTACCC TAACTCCAAG 1140
   TACGACAACA TCAAGAAGGG CATGAAC 1167
(2) INFORMATION POUR LA SEQ ID NO: 11:
   (i) CARACTERISTIQUES DE LA SEQUENCE:
      (A) LONGUEUR: 309 acides aminés
      (B) TYPE: acide aminé
      (D) CONFIGURATION: linéaire
   (ii) TYPE DE MOLECULE: protéine
   (xi) DESCRIPTION DE LA SEQUENCE: SEQ ID NO: 11:
   Ile Gly Val Cys Tyr Gly Met Leu Gly Asn Asn Leu Pro Ser Ala Asn
   1 5 10 15
   Asp Val Ile Gly Leu Tyr Arg Ser Asn Asn Ile Lys Arg Met Arg Leu
   20 25 30
   Tyr Asp Pro Asn Gln Ala Ala Leu Glu Ala Leu Arg Asn Ser Gly Ile
   35 40 45
   Glu Leu Ile Leu Gly Val Pro Asn Ser Asp Leu Gln Gly Leu Ala Thr
   50 55 60
   Asn Pro Asp Thr Ser Arg Gln Trp Val Gln Lys Asn Val Leu Asn Phe
   65 70 75 80
   Trp Pro Ser Val Lys Ile Lys Tyr Val Ala Val Gly Asn Glu Val Ser
   85 90 95
   Pro Val Gly Gly Ser Ser Ser Val Ala Gln Tyr Val Leu Pro Ala Ile
   100 105 110
   Gln Asn Val Tyr Gln Ala Ile Arg Ala Gln Gly Leu His Asp Gln Ile
   115 120 125
   Lys Val Ser Thr Ser Ile Asp Met Thr Leu Ile Gly Asn Ser Phe Pro
   130 135 140
   Pro Ser Gln Gly Ser Phe Arg Gly Asp Val Arg Ser Tyr Leu Asp Pro
   145 150 155 160
   Ile Ile Gly Tyr Leu Val Tyr Ala Asn Ala Pro Leu Leu Val Asn Val
   165 170 175
   Tyr Pro Tyr Phe Ser Tyr Thr Gly Asn Pro Arg Asp Ile Ser Leu Pro
   180 185 190
   Tyr Ala Leu Phe Thr Ala Pro Asn Val Val Val Trp Asp Gly Gln Tyr
   195 200 205
   Gly Tyr Gln Asn Leu Phe Asp Ala Met Leu Asp Ser Val His Ala Ala
   210 215 220
   Ile Asp Asn Thr Lys Ile Gly Tyr Val Glu Val Val Val Ser Glu Ser
   225 230 235 240
   Gly Trp Pro Ser Asp Gly Gly Phe Ala Ala Thr Tyr Asp Asn Ala Arg
   245 250 255
   Val Tyr Leu Asp Asn Leu Val Arg Arg Ala Asn Arg Gly Ser Pro Arg
   260 265 270
   Arg Pro Ser Lys Pro Thr Glu Thr Tyr Ile Phe Ala Met Phe Asp Glu
   275 280 285
   Asn Gln Lys Asn Pro Glu Ile Glu Lys His Phe Gly Leu Phe Asn Pro
   290 295 300
   Asn Lys Gln Lys Lys
   305
(2) INFORMATION POUR LA SEQ ID NO: 12:
   (i) CARACTERISTIQUES DE LA SEQUENCE:
      (A) LONGUEUR: 28 acides aminés
      (B) TYPE: acide aminé
      (D) CONFIGURATION: linéaire
   (ii) TYPE DE MOLECULE: peptide
   (xi) DESCRIPTION DE LA SEQUENCE: SEQ ID NO: 12:
   Tyr Pro Phe Gly Phe Gly Gly Lys Arg Leu Gly Lys Val Val Ile Asp
   1 5 10 15
   Asp Phe Asn Ala Thr Thr Ser Ile Lys Ser Asp Val
   20 25
(2) INFORMATION POUR LA SEQ ID NO: 13:
   (i) CARACTERISTIQUES DE LA SEQUENCE:
      (A) LONGUEUR: 338 acides aminés
      (B) TYPE: acide aminé
      (D) CONFIGURATION: linéaire
   (ii) TYPE DE MOLECULE: protéine
   (xi) DESCRIPTION DE LA SEQUENCE: SEQ ID NO: 13:
   Gln Ile Gly Val Cys Tyr Gly Met Leu Gly Asn Asn Leu Pro Ser Ala
   1 5 10 15
   Asn Asp Val Ile Gly Leu Tyr Arg Ser Asn Asn Ile Lys Arg Met Arg
   20 25 30
   Leu Tyr Asp Pro Asn Gln Ala Ala Leu Glu Ala Leu Arg Asn Ser Gly
   35 40 45
   Ile Glu Leu Ile Leu Gly Val Pro Asn Ser Asp Leu Gln Gly Leu Ala
   50 55 60
   Thr Asn Pro Asp Thr Ser Arg Gln Trp Val Gln Lys Asn Val Leu Asn
   65 70 75 80
   Phe Trp Pro Ser Val Lys Ile Lys Tyr Val Ala Val Gly Asn Glu Val
   85 90 95
   Ser Pro Val Gly Gly Ser Ser Ser Val Ala Gln Tyr Val Leu Pro Ala
   100 105 110
   Ile Gln Asn Val Tyr Gln Ala Ile Arg Ala Gln Gly Leu His Asp Gln
   115 120 125
   Ile Lys Val Ser Thr Ser Ile Asp Met Thr Leu Ile Gly Asn Ser Phe
   130 135 140
   Pro Pro Ser Gln Gly Ser Phe Arg Gly Asp Val Arg Ser Tyr Leu Asp
   145 150 155 160
   Pro Ile Ile Gly Tyr Leu Val Tyr Ala Asn Ala Pro Leu Leu Val Asn
   165 170 175
   Val Tyr Pro Tyr Phe Ser Tyr Thr Gly Asn Pro Arg Asp Ile Ser Leu
   180 185 190
   Pro Tyr Ala Leu Phe Thr Ala Pro Asn Val Val Val Trp Asp Gly Gln
   195 200 205
   Tyr Gly Tyr Gln Asn Leu Phe Asp Ala Met Leu Asp Ser Val His Ala
   210 215 220
   Ala Ile Asp Asn Thr Lys Ile Gly Tyr Val Glu Val Val Val Ser Glu
   225 230 235 240
   Ser Gly Trp Pro Ser Asp Gly Gly Phe Ala Ala Thr Tyr Asp Asn Ala
   245 250 255
   Arg Val Tyr Leu Asp Asn Leu Val Arg Arg Ala Asn Arg Gly Ser Pro
   260 265 270
   Arg Arg Pro Ser Lys Pro Thr Glu Thr Tyr Ile Phe Ala Met Phe Asp
   275 280 285
   Glu Asn Gln Lys Asn Pro Glu Ile Glu Lys His Phe Gly Leu Phe Asn
   290 295 300
   Pro Asn Lys Gln Lys Lys Tyr Pro Phe Gly Phe Gly Gly Lys Arg Leu
   305 310 315 320
   Gly Lys Val Val Ile Asp Asp Phe Asn Ala Thr Thr Ser Ile Lys Ser
   325 330 335
   Asp Val
(2) INFORMATION POUR LA SEQ ID NO: 14:
   (i) CARACTERISTIQUES DE LA SEQUENCE:
      (A) LONGUEUR: 32 acides aminés
      (B) TYPE: acide aminé
      (D) CONFIGURATION: linéaire
   (ii) TYPE DE MOLECULE: peptide
   (xi) DESCRIPTION DE LA SEQUENCE: SEQ ID NO: 14:
   Met Pro Ser Leu Phe Ala Arg Asn Gln Arg Phe Ser Leu Ala Thr Leu
   1 5 10 15
   Leu Leu Leu Leu Glu Leu Leu Thr Gly Asn Leu Arg Met Ala Asp Ala
   20 25 30
(2) INFORMATION POUR LA SEQ ID NO: 15:
   (i) CARACTERISTIQUES DE LA SEQUENCE:
      (A) LONGUEUR: 927 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (xi) DESCRIPTION DE LA SEQUENCE: SEQ ID NO: 15:
   ATTGGTGTGT GTTATGGCAT GCTGGGCAAC AATCTACCGT CAGCAAACGA TGTTATAGGT 60
   CTTTATAGAT CAAATAACAT AAAGAGAATG AGACTCTATG ATCCTAATCA AGCTGCTCTA 120
   GAAGCACTTA GAAATTCTGG CATTGAACTC ATTCTTGGGG TGCCAAACTC TGACCTTCAA 180
   GGCCTTGCCA CCAATCCTGA CACTTCTCGT CAATGGGTGC AAAAAAACGT GTTGAACTTT 240
   TGGCCTAGTG TCAAAATCAA GTACGTGGCA GTTGGAAATG AAGTGAGTCC CGTTGGAGGC 300
   TCTTCTTCGG TAGCCCAATA TGTTCTACCT GCCATCCAAA ATGTATACCA AGCAATAAGA 360
   GCTCAAGGCC TTCATGATCA AATCAAGGTT TCAACATCTA TTGACATGAC CCTAATAGGA 420
   AACTCTTTCC CTCCATCGCA AGGTTCCTTC AGGGGTGATG TGAGATCATA CCTAGATCCC 480
   ATAATTGGGT ACTTGGTATA TGCAAATGCA CCATTACTAG TCAATGTGTA CCCTTATTTT 540
   AGTTACACTG GTAACCCCCG TGACATATCA CTTCCCTATG CTCTTTTCAC AGCACCAAAT 600
   GTTGTGGTAT GGGATGGTCA ATATGGGTAC CAAAATTTGT TTGATGCTAT GTTGGATTCA 660
   GTACATGCAG CCATTGATAA CACTAAGATT GGTTATGTGG AGGTTGTTGT ATCCGAGAGT 720
   GGGTGGCCAT CAGATGGAGG ATTTGCTGCC ACTTATGACA ACGCACGCGT GTACTTAGAC 780
   AATTTGGTTC GTCGTGCTAA TAGAGGAAGC CCAAGAAGGC CTTCGAAGCC CACTGAGACT 840
   TATATATTTG CCATGTTCGA TGAAAATCAA AAAAATCCAG AGATAGAGAA ACATTTTGGG 900
   CTCTTCAATC CCAACAAACA AAAAAAA 927
(2) INFORMATION POUR LA SEQ ID NO: 16:
   (i) CARACTERISTIQUES DE LA SEQUENCE:
      (A) LONGUEUR: 751 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (vii) SOURCE IMMEDIATE:
      (B) CLONE: gf-2.8 de la germine de blé
   (ix) CARACTERISTIQUE ADDITIONELLE:
      (A) NOM/CLE: CDS
      (B) EMPLACEMENT: 21..692
   (xi) DESCRIPTION DE LA SEQUENCE: SEQ ID NO: 16:
   AAGCTTATTA CATAGCAAGC ATG GGG TAC TCC AAA ACC CTA GTA GCT GGC      50
                         Met Gly Tyr Ser Lys Thr Leu Val Ala Gly
                           1               5                  10
   GTG TTC GCA ATG CTG TTA CTA GCT CCG GCC GTC TTG GCC ACC GAC CCA    98
   Val Phe Ala Met Leu Leu Leu Ala Pro Ala Val Leu Ala Thr Asp Pro
                    15                  20                  25
   GAC CCT CTC CAG GAC TTC TGT GTC GCC GAC CTC GAC GGC AAG GCG GTC      146
   Asp Pro Leu Gln Asp Phe Cys Val Ala Asp Leu Asp Gly Lys Ala Val
                   30                  35                  40
   TCG GTG AAC GGG CAC ACG TGC AAG CCC ATG TCG GAG GCC GGC GAC GAC      194
   Ser Val Asn Gly His Thr Cys Lys Pro Met Ser Glu Ala Gly Asp Asp
               45                  50                  55
   TTC CTC TTC TCG TCC AAG TTG GCC AAG GCC GGC AAC ACG TCC ACC CCG      242
   Phe Leu Phe Ser Ser Lys Leu Ala Lys Ala Gly Asn Thr Ser Thr Pro
           60                  65                  70
   AAC GGC TCC GCC GTG ACG GAG CTC GAC GTG GCC GAG TGG CCC GGT ACC      290
   Asn Gly Ser Ala Val Thr Glu Leu Asp Val Ala Glu Trp Pro Gly Thr
    75                  80                  85                  90
   AAC ACG CTG GGT GTG TCC ATG AAC CGC GTG GAC TTT GCT CCC GGA GGC      338
   Asn Thr Leu Gly Val Ser Met Asn Arg Val Asp Phe Ala Pro Gly Gly
                       95                 100                 105
   ACC AAC CCA CCA CAC ATC CAC CCG CGT GCC ACC GAG ATC GGC ATC GTG      386
   Thr Asn Pro Pro His Ile His Pro Arg Ala Thr Glu Ile Gly Ile Val
                   110                 115                 120
   ATG AAA GGT GAG CTT CTC GTG GGA ATC CTT GGC AGC CTC GAC TCC GGG      434
   Met Lys Gly Glu Leu Leu Val Gly Ile Leu Gly Ser Leu Asp Ser Gly
               125                 130                 135
   AAC AAG CTC TAC TCG AGG GTG GTG CGC GCC GGA GAG ACG TTC CTC ATC      482
   Asn Lys Leu Tyr Ser Arg Val Val Arg Ala Gly Glu Thr Phe Leu Ile
           140                 145                 150
   CCA CGG GGC CTC ATG CAC TTC CAG TTC AAC GTC GGT AAG ACC GAG GCC      530
   Pro Arg Gly Leu Met His Phe Gln Phe Asn Val Gly Lys Thr Glu Ala
   155                 160                 165                 170
   TCC ATG GTC GTC TCC TTC AAC AGC CAG AAC CCC GGC ATT GTC TTC GTG      578
   Ser Met Val Val Ser Phe Asn Ser Gln Asn Pro Gly Ile Val Phe Val
                           175                 180                 185
   CCC CTC ACG CTC TTC GGC TCC AAC CCG CCC ATC CCA ACG CCG GTG CTC      626
   Pro Leu Thr Leu Phe Gly Ser Asn Pro Pro Ile Pro Thr Pro Val Leu
                       190                 195                 200
   ACC AAG GCA CTC CGG GTG GAG GCC AGG GTC GTG GAA CTT CTC AAG TCC      674
   Thr Lys Ala Leu Arg Val Glu Ala Arg Val Val Glu Leu Leu Lys Ser
                   205                 210                 215
   AAG TTT GCC GCT GGG TTT TAATTTCTAG GAGCCTTCCC TGAAATGATA             722
   Lys Phe Ala Ala Gly Phe
       220
   ATTATATAAT TCCATATATG CATGCTAGC                                      751
(2) INFORMATION POUR LA SEQ ID NO: 17:
   (i) CARACTERISTIQUES DE LA SEQUENCE:
      (A) LONGUEUR: 224 acides aminés
      (B) TYPE: acide aminé
      (D) CONFIGURATION: linéaire
   (ii) TYPE DE MOLECULE: protéine
   (xi) DESCRIPTION DE LA SEQUENCE: SEQ ID NO: 17:
   Met Gly Tyr Ser Lys Thr Leu Val Ala Gly Val Phe Ala Met Leu Leu
     1               5                  10                  15
   Leu Ala Pro Ala Val Leu Ala Thr Asp Pro Asp Pro Leu Gln Asp Phe
               20                  25                  30
   Cys Val Ala Asp Leu Asp Gly Lys Ala Val Ser Val Asn Gly His Thr
           35                  40                  45
   Cys Lys Pro Met Ser Glu Ala Gly Asp Asp Phe Leu Phe Ser Ser Lys
       50                  55                  60
   Leu Ala Lys Ala Gly Asn Thr Ser Thr Pro Asn Gly Ser Ala Val Thr
   65 70 75 80
   Glu Leu Asp Val Ala Glu Trp Pro Gly Thr Asn Thr Leu Gly Val Ser
   85 90 95
   Met Asn Arg Val Asp Phe Ala Pro Gly Gly Thr Asn Pro Pro His Ile
   100 105 110
   His Pro Arg Ala Thr Glu Ile Gly Ile Val Met Lys Gly Glu Leu Leu
   115 120 125
   Val Gly Ile Leu Gly Ser Leu Asp Ser Gly Asn Lys Leu Tyr Ser Arg
   130 135 140
   Val Val Arg Ala Gly Glu Thr Phe Leu Ile Pro Arg Gly Leu Met His
   145 150 155 160
   Phe Gln Phe Asn Val Gly Lys Thr Glu Ala Ser Met Val Val Ser Phe
   165 170 175
   Asn Ser Gln Asn Pro Gly Ile Val Phe Val Pro Leu Thr Leu Phe Gly
   180 185 190
   Ser Asn Pro Pro Ile Pro Thr Pro Val Leu Thr Lys Ala Leu Arg Val
   195 200 205
   Glu Ala Arg Val Val Glu Leu Leu Lys Ser Lys Phe Ala Ala Gly Phe
   210 215 220
(2) INFORMATION POUR LA SEQ ID NO: 18:
   (i) CARACTERISTIQUES DE LA SEQUENCE:
      (A) LONGUEUR: 38 acides aminés
      (B) TYPE: acide aminé
      (D) CONFIGURATION: linéaire
   (ii) TYPE DE MOLECULE: peptide
   (xi) DESCRIPTION DE LA SEQUENCE: SEQ ID NO: 18:
   Thr Asp Pro Asp Pro Leu Gln Asp Phe Xaa Val Ala Asp Leu Asp Gly
   1 5 10 15
   Lys Ala Val Ser Val Asn Gly His Thr Xaa Lys Pro Met Ser Glu Ala
   20 25 30
   Gly Asp Asp Phe Leu Phe
   35
(2) INFORMATION POUR LA SEQ ID NO: 19:
   (i) CARACTERISTIQUES DE LA SEQUENCE:
      (A) LONGUEUR: 9 acides aminés
      (B) TYPE: acide aminé
      (D) CONFIGURATION: linéaire
   (ii) TYPE DE MOLECULE: peptide
   (xi) DESCRIPTION DE LA SEQUENCE: SEQ ID NO: 19:
   Ala Gly Glu Thr Phe Val Ile Pro Arg
   1 5
(2) INFORMATION POUR LA SEQ ID NO: 20:
   (i) CARACTERISTIQUES DE LA SEQUENCE:
      (A) LONGUEUR: 10 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (xi) DESCRIPTION DE LA SEQUENCE: SEQ ID NO: 20:
   AGCTGGATCC 10